(19) Europäisches Patentamt
European Patent Office
Office européen des brevets

(11) EP 2 546 344 A2

(12) EUROPEAN PATENT APPLICATION

(43) Date of publication:
**16.01.2013 Bulletin 2013/03**

(51) Int Cl.:
***C12N 15/09*** *(2006.01)* ***C12Q 1/68*** *(2006.01)*

(21) Application number: **12188125.4**

(22) Date of filing: **03.02.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **08.02.2008 JP 2008029460**

(62) Document number(s) of the earlier application(s) in accordance with Art. 76 EPC:
**09707584.0 / 2 251 422**

(71) Applicant: **Wako Pure Chemical Industries, Ltd.**
**Osaka-shi,**
**Osaka 540-8605 (JP)**

(72) Inventors:
- **Ishikawa, Tomokazu**
  **Hyogo, Hyogo 661-0963 (JP)**
- **Wada, Koichiro**
  **Okayama, Okayama 700-8558 (JP)**
- **Kumon, Hiromi**
  **Okayama, Okayama 700-0955 (JP)**

(74) Representative: **von Kreisler Selting Werner**
**Deichmannhaus am Dom**
**Bahnhofsvorplatz 1**
**50667 Köln (DE)**

Remarks:
This application was filed on 15-10-2012 as a divisional application to the application mentioned under INID code 62.

# (54) Primer and probe for detecting chlamydophilia caviae, as well as chlamydophilia caviae detection method using the same

(57) Provided are a novel primer and a probe for the detection of *Chlamydophila caviae* which can exclude any false positive result for the diagnosis, as well as a method for detecting *Chlamydophila caviae* more simply, rapidly and with high accuracy using the same.

According to the method for detection of *Chlamydophila caviae* using the primer and/or the probe of the present invention, the detection of *Chlamydophila caviae* can be performed more rapidly and with high precision compared with a conventional bacterial species identification method performed by culture examination and the like. In addition, by using the detection method of the present invention, the *Chlamydophila caviae* itself can also be quantified.



Printed by Jouve, 75001 PARIS (FR)

**Description**

TECHNICAL FIELD

The present invention relates to a method for detection and/or identifying *Chlamydophila caviae* through nucleic acid amplification and detection system.

BACKGROUND ART

In concurrence with diversification of the sexual manners and customs and change in pattern of sexual behavior of the Japanese centering on the young people, an increase in the chlamydial infection as a sexually transmitted disease is significant today.

*Chlamydia* is an obligate intracellular parasitic bacterium of eukaryotic cell. It grows proliferously in a host cell, and forms an inclusion body in the cytoplasm of the cell. This nature causes clinical symptoms to the host. For example, the causative microorganism of genital chlamydial infection is *Chlamydia trachomatis,* which mainly develops the symptoms of urethritis in a man and cervicitis in a woman. In addition, although *Chlamydophila caviae* is a causative microorganism of the inclusion body conjunctivitis (GPIC) of a guinea pig, pathogenesis to humans has not been determined till today.

On the other hand, it has been suggested by Hiromi Kumon, et al. of Okayama University that, in the chlamydiae which caused infection in man, a chlamydia other than *Chlamydia trachomatis* may exist, and that such other chlamydia which infects human may possibly be *Chlamydophila caviae* (Non-patent Literature 1).

Although the *Chlamydophila caviae* was dealt with as *Chlamidya psittaci* GPIC isolate till 1999, it became an independent species according to the classification reorganization of *Chlamidia* genus based on genomic analysis in recent years. In addition, *Chlamydophila caviae* is the 4th species which the analysis of whole genome has been completed in *Chlamydiaceae* family (Non-patent Literature 2), and comparative analysis especially with *Chlamydia pneumoniae* which infects human widely has been carried out.

As for a method of detecting chlamydia, methods of detecting antigen, such as direct fluorescent antibody staining (DFA), enzyme immunoassay (EIA), and enzyme linked immunosorbent assay (ELISA), have been developed. In addition, a method for detection of *Chlamydia trachomatis* by probe hybridization technique using labeling-substance-labeled single-stranded DNA which is complementary to the ribosomal RNA of *Chlamydia trachomatis,* has also been developed (Non-patent Literature 3).

On the other hand, as a higher sensitive detection method, DNA amplification technology such as polymerase chain reaction (PCR), ligase chain reaction (LCR), and standard displacement amplification (SDA), have been developed. For example, Domeika et al. (Non-patent Literature 4), Bauwens et al. (Non-patent Literature 5), and specification of U.S. Patent No. 5,232,829 (Patent Literature 1) have reported a method for detection of *Chlamydia trachomatis* by carrying out the PCR and subsequent micro titration and plate hybridization. In addition, a method for detection of *Chlamydia trachomatis* by carrying out the LCR and subsequent microparticle sandwich immunoassay detection has also been reported (Non-patent Literature 6, Non-patent Literature 7, and Non-patent Literature 8). However, these detection methods require 4 to 6 hours to complete the detection.

By the way, for the detection of *Chlamydophila caviae,* a fluorescence staining method and a nested PCR method (conventional PCR method) have been carried out mainly. Among them, the fluorescence staining method is a method in which, after a sample is inoculated into the cells in culture which serves as a host of *Chlamydophila caviae* such as McCoy cell and HeLa cell, the cells are treated with a specific antibody for the *Chlamydiaceae* species, then the cells are observed. This method takes about 3 days for detection, and its sensitivity and specificity are also low.

In addition, since the nested PCR method has a relatively low sensitivity, to perform the PCR, it is necessary to extract DNA after the culture of the chlamydia cell by culture to a certain amount. However, as described above, to culture the chlamydia, it is necessary to inoculate the sample into the cells in culture which serves as a host of *Chlamydophila caviae* such as McCoy cell and HeLa cell, and it is a tough work and requires about 48 to 72 hours. Therefore, at least about 3 days from culture to the detection is needed, and the specificity of detection is not satisfiable.

DNA amplification technology, such as PCR, LCR, and SDA, is a technology which is utilized widely in many fields at the present day. Nevertheless, as described above, the genetic test which involves application of these methods for detecting *Chlamydophila caviae* specifically has not been established until now. Whereas, because it has been suggested, as described above, that *Chlamydophila caviae* may have a potential to infect humans, the development of a method which can detect *Chlamydophila caviae* simply yet specifically has been desired. This is the current situation.

Non-patent Literature 1: "Properties of chlamydia separated from the affected area of cervicitis", Pathogenic Microbe Detection Information Monthly Report (IASR), August, 2004, vol. 25, No.8, p.204-205, Infectious Disease Surveillance Center, National Institute of Infectious Diseases;

Non-patent Literature 2: Read T.D. et al., Nucleic Acid Research, 2003, 31, 2134-2147;
Non-patent Literature 3: Warren R., et al., Journal of Clinical Microbiology, 1993, 31, 1663-1666;
Non-patent Literature 4: Domeika M. et al., Journal of Clinical Microbiology, 1994, 32, 2350-2352;
Non-patent Literature 5: Bauwens J.E. et al., Journal of Clinical Microbiology, 1993, 31, 3013-3106;
Non-patent Literature 6: Chernesky Max A. et al., Journal of Clinical Microbiology, 1994, 32, 2682-2685;
Non-patent Literature 7: Lee H.H. et al., Lancet, 1995, 345, 213-216;
Non-patent Literature 8: Bassiri M. et al., Journal of Clinical Microbiology, 1995, 33,898-900;
Non-patent Literature 9: F. Poly et al., J. Bacteriology, 2004, 186 (14), p.4781-4795; Patent Literature 1: U.S. Patent No.5,232,829

DISCLOSURE OF INVENTION

PROBLEMS TO BE SOLVED BY THE INVENTION

The present invention was made in view of the above-described situation, and an object of the present invention is to provide a new primer for the detection of *Chlamydophila caviae* which can exclude any false-positive result for the diagnosis; and to provide a method for detection of *Chlamydophila caviae* more simply, rapidly and with high accuracy.

MEANS FOR SOLVING THE PROBLEM

The present invention was made for the purpose of solving the above-described problems, and comprises the following composition:

(1) An oligonucleotide which comprises a part or the entire of a nucleotide sequence selected from SEQ ID NO:1, SEQ ID NO:2, SEQ ID NO:3, SEQ ID NO:4, SEQ ID NO:5 and SEQ ID NO:6 (wherein, that the characters of A, C, G and T represent adenine, cytosine, guanine and thymine, respectively; and, T at arbitrary position may be replaced by uracil (U); and hereinafter, same as above), or a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO: 6, and which is capable of hybridizing with the nucleotide sequence for a *Chlamydophila caviae* gene.
(2) A primer for detection of *Chlamydophila caviae* comprising an oligonucleotide which comprises a part or the entire of a nucleotide sequence selected from SEQ ID NO:1, SEQ ID NO:2, SEQ ID NO:3, SEQ ID NO:4, SEQ ID NO:5 and SEQ ID NO:6, or a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6, and which is capable of hybridizing with the nucleotide sequence for a *Chlamydophila caviae* gene.
(3) A probe for detection of *Chlamydophila caviae* comprising an oligonucleotide which comprises a part or the entire of a nucleotide sequence selected from SEQ ID NO:1, SEQ ID NO:2, SEQ ID NO:3, SEQ ID NO:4, SEQ ID NO:5 and SEQ ID NO:6, or a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6, and is capable of hybridizing with the nucleotide sequence of a *Chlamydophila caviae* gene.
(4) A method for detection of *Chlamydophila caviae,* characterized in that an oligonucleotide which comprises a part or the entire of a nucleotide sequence selected from SEQ ID NO:1, SEQ ID NO:2, SEQ ID NO:3, SEQ ID NO:4, SEQ ID NO:5 and SEQ ID NO:6, or a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6, and which is capable of hybridizing with the nucleotide sequence of a *Chlamydophila caviae* gene is used as a primer and/or a probe.
(5) A reagent kit for detection of *Chlamydophila caviae* comprising an oligonucleotide which comprises a part or the entire of a nucleotide sequence selected from SEQ ID NO:1, SEQ ID NO:2, SEQ ID NO:3, SEQ ID NO:4, SEQ ID NO:5 and SEQ ID NO:6, or a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6, and which is capable of hybridizing with the nucleotide sequence of a *Chlamydophila caviae* gene as a primer and/or a probe.

The present inventor conducted theoretical verification and experimental verification of genetic homology between species with regard to the nucleotide sequence of various species including *Chlamydophila caviae* and other living organisms. As a result, the present inventor have found that a nucleotide sequence is present in the nucleic acid fragments derived from *Chlamydophila caviae* obtained by the method using microarray technique, which is capable of hybridizing specifically with the nucleotide sequence of a *Chlamydophila caviae* gene and is useful for detection of *Chlamydophila caviae.*
And so, on the basis of these findings, the present inventor further studied intensively and obtained oligonucleotides specific for *Chlamydophila caviae* (the nucleotide sequence shown in SEQ ID NO:1, SEQ ID NO:2, SEQ ID NO:3, SEQ ID NO:4, SEQ ID NO:5 and SEQ ID NO:6), and has found that these nucleotide sequences are useful for detection of *Chlamydophila caviae.* And further, on the basis of these sequences, a primer and a probe for the detection of *Chlamydophila caviae* have been developed, and thus a method for detection of *Chlamydophila caviae* using these primer and probe has been established.

EFFECT OF THE INVENTION

According to the method for detection of *Chlamydophila caviae* using the primer and/or the probe of the present invention, *Chlamydophila caviae* can be detected more rapidly and with high accuracy as compared to the conventional bacterium identification method by bacterial cell culture examination and the like. In addition, by carrying out the detection using the method of the present invention, *Chlamydophila caviae* cell can also be quantified.

Furthermore, a high specificity of not reactive to other chlamydiae which cause infection in human could also be realized.

As it is anticipated that a need of epidemiological investigation and studies on the possibility of causing urethritis and cervicitis by the infection of *Chlamydophila caviae* in human will increase from now on, and therefore, contribution of the present invention to this industry will be great.

BRIEF DESCRIPTION OF THE DRAWINGS

Fig. 1 shows the results of detection obtained by the real-time PCR in Example 3, which is a standard curve drawn by plotting Ct value (Y-axis) for the copy number of genome (X-axis, logarithmic scale) of each DNA sample for PCR.

BEST MODE FOR CARRYING OUT THE INVENTION

In the present invention, *Chlamydophila caviae* gene refers to an arbitral unit of nucleotide sequence (a region) in the whole genome sequence owned by *Chlamydophila caviae*.

The oligonucleotide of the present invention includes an oligonucleotide which comprises a part or an entire of a nucleotide sequence selected from SEQ ID NO:1, SEQ ID NO:2, SEQ ID NO:3, SEQ ID NO:4, SEQ ID NO:5 and SEQ ID NO:6, or a part or an entire of a sequence complementary to the nucleotide sequence selected from SEQ ID NO:1, SEQ ID NO:2, SEQ ID NO:3, SEQ ID NO:4, SEQ ID NO:5 and SEQ ID NO:6, and which is capable of hybridizing with the nucleotide sequence of a *Chlamydophila caviae* gene (hereinafter, obtically designated as "the oligonucleotide of the present invention").

An oligonucleotide involved in the present invention which comprises a part or the entire of a nucleotide sequences selected from SEQ ID NO:1, SEQ ID NO:2, SEQ ID NO:3, SEQ ID NO:4, SEQ ID NO:5 and SEQ ID NO:6 includes, for example,

(1) an oligonucleotide comprising a nucleotide sequence having a sequence homology of not less than 70%, preferably not less than 80%, more preferably not less than 90%, further more preferably not less than 95% to the nucleotide sequence selected from SEQ ID NO:1, SEQ ID NO:2, SEQ ID NO:3, SEQ ID NO:4, SEQ ID NO:5 and SEQ ID NO:6, or

(2) an oligonucleotide characterized by comprising not less than 10 consecutive nucleotides, preferably not less than 15 consecutive nucleotides, more preferably not less than 20 consecutive nucleotides in a sequence selected from SEQ ID NO:1, SEQ ID NO:2, SEQ ID NO:3, SEQ ID NO:4, SEQ ID NO:5 and SEQ ID NO:6, or the like.

A specific example of oligonucleotide involved in the present invention which comprises the entire of a nucleotide sequences selected from SEQ ID NO:1, SEQ ID NO:2, SEQ ID NO:3, SEQ ID NO:4, SEQ ID NO:5 and SEQ ID NO:6 includes, for example, an oligonucleotide consisting of a nucleotide sequence selected from SEQ ID NO:1, SEQ ID NO: 2, SEQ ID NO:3, SEQ ID NO:4, SEQ ID NO:5 and SEQ ID NO:6, or an oligonucleotide comprising a nucleotide sequence selected from SEQ ID NO:1, SEQ ID NO:2, SEQ ID NO:3, SEQ ID NO:4, SEQ ID NO:5 and SEQ ID NO:6.

A specific example of the oligonucleotide comprising a part of a nucleotide sequence selected from SEQ ID NO:1, SEQ ID NO:2, SEQ ID NO:3, SEQ ID NO:4, SEQ ID NO:5 and SEQ ID NO:6 includes, for example, the one which comprises a part or an entire of a sequence selected from the nucleotide sequences shown in SEQ ID NO:7 to 39.

A specific example of the oligonucleotide comprising a part of a nucleotide sequence shown in SEQ ID NO:1 includes, for example, the one which comprises a part or the entire of a sequence selected from SEQ ID NO:7 to 10 and SEQ ID NO:29 to 30.

A specific example of the oligonucleotide comprising a part of a nucleotide sequence shown in SEQ ID NO:2 includes, for example, the one which comprises a part or the entire of a sequence selected from SEQ ID NO:11 to 14 and SEQ ID NO:31 to 32.

A specific example of the oligonucleotide comprising a part of a nucleotide sequence shown in SEQ ID NO:3 includes, for example, the one which comprises a part or the entire of a sequence selected from SEQ ID NO:15 to 18 and SEQ ID NO:33 to 34.

A specific example of the oligonucleotide comprising a part of a nucleotide sequence shown in SEQ ID NO:4 includes, for example, the one which comprises a part or the entire of a sequence selected from SEQ ID NO:19 to 22 and SEQ ID NO:35 to 36.

A specific example of the oligonucleotide comprising a part of a nucleotide sequence shown in SEQ ID NO:5 includes, for example, the one which comprises a part or the entire of a sequence selected from SEQ ID NO:23 to 26 and SEQ ID NO:37 to 38.

A specific example of the oligonucleotide comprising a part of a nucleotide sequence shown in SEQ ID NO:6 includes, for example, the one which comprises a part or the entire of a sequence selected from SEQ ID NO:27 to 28 and SEQ ID NO:39.

A specific example of the oligonucleotide comprising the entire of a nucleotide sequence selected from SEQ ID NO:7 to 39 includes an oligonucleotide consisting of a nucleotide sequence selected from SEQ ID NO:7 to 39, or an oligonucleotide comprising a nucleotide sequence selected from SEQ ID NO:7 to 39.

In addition, an example of the oligonucleotide comprising a part of a nucleotide sequence selected from SEQ ID NO:7 to 39 includes an oligonucleotide which comprises more than 10 consecutive nucleotides, preferably more than 15 consecutive nucleotides in the nucleotide sequence selected from SEQ ID NO:7 to 39.

The oligonucleotide involved in the present invention which comprises a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6 includes, for example, an oligonucleotide comprising a part or the entire of a nucleotide sequence which is capable of hybridizing with an oligonucleotide consisting of a nucleotide sequence selected from SEQ ID NO:1, SEQ ID NO:2, SEQ ID NO:3, SEQ ID NO:4, SEQ ID NO:5 and SEQ ID NO:6 of the present invention, and the like.

The oligonucleotide comprising a part or the entire of a nucleotide sequence which is capable of hybridizing with an oligonucleotide consisting of a nucleotide sequence selected from SEQ ID NO:1, SEQ ID NO:2, SEQ ID NO:3, SEQ ID NO:4, SEQ ID NO:5 and SEQ ID NO:6 of the present invention includes, specifically, for example, an oligonucleotide comprising a part or the entire of a nucleotide sequence which is capable of hybridizing under high stringent condition or stringent condition with an oligonucleotide consisting of a nucleotide sequence selected from SEQ ID NO: 1, SEQ ID NO:2, SEQ ID NO:3, SEQ ID NO:4, SEQ ID NO:5 and SEQ ID NO:6 of the present invention, and the like.

The phrase of "high stringent condition" used herein means, specifically, for example, "the condition where hybridization is carried out in 50% formamide at 42°C to 70°C, preferably 60°C to 70°C, and followed by washing with 0.2 to 2 x SSC containing 0.1% sodium dodecyl sulfate (SDS) at 25°C to 70°C".

In addition, the phrase of "stringent condition" means, specifically, for example, "the condition where hybridization is carried out in 6 x SSC or a hybridization solution with equivalent salt concentration at the temperature of 50°C to 70°C for 16 hours, and then, if needed, pre-washing with 6 x SSC or a solution with the equivalent salt concentration, and followed by washing with 1 x SSC or a solution with the equivalent salt concentration and the like".

An example of the oligonucleotide comprising a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6 involved in the present invention includes, for example,

(1) an oligonucleotide comprising a nucleotide sequence having a sequence homology of not less than 70%, preferably not less than 80%, more preferably not less than 90%, further more preferably not less than 95% to the sequence complementary to the nucleotide sequence selected from SEQ ID NO:1, SEQ ID NO:2, SEQ ID NO:3, SEQ ID NO:4, SEQ ID NO:5 and SEQ ID NO:6, or

(2) an oligonucleotide characterized by comprising more than 10 consecutive nucleotides, preferably more than 15 nucleotides, more preferably more than 20 nucleotides in the sequence complementary to the nucleotide sequence selected from SEQ ID NO:1, SEQ ID NO:2, SEQ ID NO:3, SEQ ID NO:4, SEQ ID NO:5 and SEQ ID NO:6, and the like.

A specific example of the oligonucleotide comprising the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6 involved in the present invention includes, for example, an oligonucleotide consisting of a sequence complementary to the nucleotide sequence selected from SEQ ID NO:1, SEQ ID NO:2, SEQ ID NO:3, SEQ ID NO:4, SEQ ID NO:5 and SEQ ID NO:6, or an oligonucleotide comprising a sequence complementary to the nucleotide sequence selected from SEQ ID NO:1, SEQ ID NO:2, SEQ ID NO:3, SEQ ID NO:4, SEQ ID NO:5 and SEQ ID NO:6.

A specific example of the oligonucleotide comprising a part of a sequence complementary to the nucleotide sequence selected from SEQ ID NO:1, SEQ ID NO:2, SEQ ID NO:3, SEQ ID NO:4, SEQ ID NO:5 and SEQ ID NO:6 includes, for example, an oligonucleotide comprising a part or the entire of a sequence complementary to the nucleotide sequence selected from SEQ ID NO:7 to 39.

A specific example of the oligonucleotide comprising the entire of a sequence complementary to the nucleotide sequence selected from SEQ ID NO:7 to 39 includes, for example, an oligonucleotide consisting of a sequence complementary to the nucleotide sequence selected from SEQ ID NO:7 to 39, or an oligonucleotide comprising a sequence complementary to the nucleotide sequence selected from SEQ ID NO:7 to 39.

In addition, an example of the oligonucleotide comprising a part of a sequence complementary to the nucleotide sequence selected from SEQ ID NO:7 to 39 includes an oligonucleotide which comprises more than 10 consecutive nucleotides, preferably more than 15 consecutive nucleotides in a sequence complementary to the nucleotide sequence selected from SEQ ID NO:7 to 39.

The oligonucleotide which is capable of hybridizing with the nucleotide sequence of a *Chlamydophila caviae* gene involved in the present invention includes an oligonucleotide having a nucleotide sequence capable of hybridizing with the nucleotide sequence of a *Chlamydophila caviae* gene under a high stringent condition or a stringent condition, and the like. The high stringent condition and the stringent condition are as having described above.

It should be noted that, the oligonucleotide of the present invention may be either deoxyribonucleic acid (DNA) or ribonucleic acid (RNA). In the case of ribonucleic acid, it goes without saying that thymidine residue (T) may be read as uridine (U) residue. In addition, it may be a DNA comprising uridine residue which is synthesized by exchanging T at arbitral position by U. Also, it may be an RNA comprising thymidine residue which is synthesized by exchanging U at arbitral position by T. In addition, there may be deletion, insertion or replacement of one or plural nucleotides. One or plural nucleotides may be a modified nucleotide such as inosine (I).

The method for obtaining an oligonucleotide of the present invention includes, but not limited to, for example, a method for preparation by chemical synthesis well known per se. In this method, it is possible to obtain an oligonucleotide of the same quality without difficulty in larger scale at lower cost compared to the method of obtaining an oligonucleotide or a polynucleotide by genetic engineering technique using a vector and the like (cloning method).

For example, by a conventional method of DNA synthesis using a DNA synthesizer, an oligonucleotide is synthesized according to the conventional phosphoramidite method, and then purified through anion exchange column chromatography. And thus, an objective oligonucleotide of the present invention can be obtained.

Alternatively, using vendor's custom service of contract synthesis, the oligonucleotide may be purchased from the vendor.

As a method for searching (screening) an oligonucleotide which can attain the purpose of the present invention, there is a subtraction method, which has been described in FEMS Microbiology Letters 166: 63-70, 1998 or Systematic and Applied Microbiology 24: 109-112, 2001, etc., namely, a method of concentrating candidate sequence by removing fragments from a group of fragments derived from the target genomic DNA, which react with a group of fragments of genomic DNA derived from species to be differentiated.

In addition, an approach in which differential display of amplification products from a target genomic DNA and a genomic DNA derived from species to be differentiated are prepared, that is, a methodology utilizing the arbitrarily primed polymerase chain reaction (AP-PCR) (JP-A-11-155589) and the like can be considered.

Further, also by use of so called microarray method, searching of an oligonucleotide which can attain the purpose of the present invention can also be performed, and the oligonucleotide of the present invention can be obtained. The brief description of the method is as follows:

Namely, for example, a shotgun clone of genomic DNA derived from *Chlamydophila caviae* is prepared, and then the DNA is purified from the obtained shotgun clone. Subsequently, the purified DNA derived from the shotgun clone is amplified by the PCR and the like, and the amplified DNA is arranged on a slide glass by common procedure to prepare a microarray. On the side, a group of fluorescent labeled genomic DNA fragments (Label-1) is prepared from the genomic DNA derived from *Chlamydophila caviae* which is a detection terget. On the other hand, a group of fluorescent labeled DNA fragments (Label-2) is prepared separately from the genomic DNA derived from species to be differentiated. And, the reactivity (binding capacity) of each Label-1 and Label-2 to the purified DNA on the microarray is assayed by carrying out a competitive hybridization method using the Label-1 and Label-2 in the same reaction system. By this assay, the candidate sequence group which react more specifically to the fragments group (Label-1) prepared with genomic DNA from the target *Chlamydophila caviae* can be selected (see, for example, Non-Patent Literature 9, etc.).

By the method described above, the oligonucleotide which hybridizes specifically with an target nucleotide sequence of a *Chlamydophila caviae* gene can be sorted out. An example of the method for selection of the oligonucleotide of the present invention using the microarray method will be described in detail below.

(1) Preparation of purified genomic DNA derived from *Chlamydophila caviae* First, a purified genomic DNA derived from *Chlamydophila caviae* is obtained. For example, the DNA may be extracted and purified from a strain of *Chlamydophila caviae* by common procedures. Alternatively, using vendor's custom service of contract extraction and purification of the genomic DNA from a strain of *Chlamydophila caviae,* the genomic DNA may be obtained from the vendor.

(2) Preparation of Whole Genome Shotgun library

As an example of the method for preparing Whole Genome Shotgun library of *Chlamydophila caviae,* a method modified from the Whole Genome Shotgun method described in Venter et al., Science 2001 Feb 16; 291 (5507): 1304-1351 will be described below.

First, the purified genomic DNA derived from *Chlamydophila caviae* obtained in the above-described (1) is diluted with an appropriate buffer solution and the like, and then shared into fragments, for example, in the presence of 20% concentration of glycerol, by treating for about 1 minute to 15 minutes using a nebulizer under a pressure of 5 kPa to 9 kPa. By this treatment, the objective size of 500 bp to 1,000 bp fraction (DNA fragment) can be recovered efficiently. The fraction obtained is purified using a commercially available extraction column.

After that, the obtained fraction (DNA fragments, containing the objective DNA fragments) is inserted into a vector DNA by ligation according to the common procedures, and thus, the recombinant DNA (Whole Genome Shotgun library of *Chlamydophila caviae*) is obtained.

The vector to be used for this purpose includes, in the case where the host cell for subsequent transformation is *E. coli,* for example, the vectors such as pBS (e.g., pBSII sk$^+$ vector (manufactured by Stratagene Corporation)), pQE-TRI plasmid (manufactured by Qiagen K.K.), pBluescript, pET, pGEM-3Z, pGEX and the like. Depending on the kind of the vector to be used, prior to the ligation, terminal of the DNA fragments may be blunted by treating with DNA polymerase and the like in advance.

Subsequently, using the obtained recombinant DNA, an appropriate host cell is transformed to obtain a transformant.

The host cell to be used for this purpose includes, for example, *Escherichia coli,* preferably JM109, DH5$\alpha$, TOP10 and the like. In addition to these, competent cells having higher transfection efficiency for the plasmid and the phage DNA may be used. For example, *E. coli* JM109 Competent Cells (manufactured by Takara Bio Inc.) and the like are included.

The transformation of the host cell may be carried out according to the common method (for example, the D. M. Morrison's method (Method in Enzymology, 68, 326-331, 1979) and the like). In addition, when a commercially available competent cell is used, the transformation may be carried out according to the protocol provided for the product.

The method for selection of the transformant which has been transformed with "the recombinant DNA having an objective DNA fragment" may be carried out, for example, by a method utilizing the property of the vector used for transformation. For example, when a vector comprising ampicillin-resistant gene is used, by culturing the transformant on a medium containing ampicillin, and followed by selecting the obtained clone, the transformant which has been transformed by the recombinant DNA having the objective DNA fragment (Whole Genome Shotgun clone Library derived from genomic DNA of *Chlamydophila caviae*) can be obtained easily.

(3) Preparation of microarray

Microarray is prepared by the following method.

Namely, from the Library of transformant (Whole Genome Shotgun clone Library derived from genomic DNA of *Chlamydophila caviae*) obtained in the above-described (2), DNA is purified according to the conventional procedures. Using the purified DNA as a template, and using a suitable primer (it may be a commercially available primer; for example, M13 Primer M1 (manufactured by Takara Bio Inc.) and M13 Primer RV (manufactured by Takara Bio Inc.) and the like), the PCR is carried out according to the conventional procedure and the obtained PCR amplification product is purified. Subsequently, according to the conventional procedures, the purified PCR amplification product is spotted on a slide glass for microarray. The spots are irradiated with UV light (60 mJ/cm$^2$ to 300 mJ/cm$^2$) to fix the PCR amplification product (comprising target genomic DNA derived from *Chlamydophila caviae*) on the slide glass, and thus the microarray is prepared.

(4) Labeling of target genomic DNA with fluorescent dye

i) Labeling of the target genomic DNA with fluorescent dye

For example, by the conventional method such as indirect labeling method using hexylamino-UTP, for example, the purified genomic DNA derived from *Chlamydophila caviae* obtained by the above-described method (1) is labeled with a labeling substance. In addition, genomic DNA as a control (for example, a chlamydia other than *Chlamydophila caviae* such as *Chlamydia psttaci,* and so on) is labeled with a different labeling substance from that used for labeling the purified genomic DNA derived from *Chlamydophila caviae.*

Labeling substance to be used for labeling the DNA includes the labeling substances usually used in this field, and widely used labeling substances include Cy3 (product name of Amersham Biosciences K.K.), Cy5 (product name of Amersham Biosciences K.K.), Alexa555 (product name of Invitrogen Corp.), Alexa647 (product name of Invitrogen Corp.) and the like.

For example, the method for labeling the DNA using Cy3 and Cy5 includes an indirect labeling method which has been modified from a protocol published by DeRisi Laboratory (www.microarray.org). In this method, at first, by carrying out an enzymatic extension reaction, a DNA chain which has been incorporated with a $\alpha$UTP having an amino group into the molecule is produced. And, to this amino group of the DNA, a fluorescent dye (succinimide body) is coupled chemically, thereby, the DNA is labeled.

That is, at first, the starting material (purified genomic DNA derived from *Chlamydophila caviae* or genomic DNA for control) is subjected to heat denaturation treatment according to the conventional procedures. After that, to the heat denatured material, 2 $\mu$L of DTT, a mixed solution of dATP/dCTP/dGTP, dTTP, Ha-dUTP and Klenow enzyme are added, and the extension reaction is carried out at 37°C for about 3 hours. The obtained reaction product is placed onto an ultrafiltration column and centrifuged at 14,000 rpm for about 4 minutes, and the concentrated solution is recovered in a microtube, and then dried thoroughly using a centrifugal vacuum drier and the like. After that, to the dried above reaction product, $NaHCO_3$ is added and mixed, and then left standing at ambient temperature for 2 to 3 minutes.

Separately, a solution of Cy3 (or Cy5) dissolved in DMSO (Cy-dye Solution Cy3, Cy-dye Solution Cy5) is prepared. This Cy-dye Solution Cy3 is added to the above-described reaction product obtained by the use of DNA derived from genome for control. Also, the Cy-dye Solution Cy5 is added to the above-described reaction product obtained by the use of genomic DNA derived from *Chlamydophila caviae.* Each mixture is incubated under light shielding at 40°C for about 60 minutes. Further, each reaction product is added with 4 M $NH_2OH$ and mixed, and incubated under light shielding for about 15 minutes to obtain labeled product of each genomic DNA. After that, the obtained labeled product is placed onto an ultrafiltration column and centrifuged at 14,000 rpm for about 4 minutes. The concentrated solution is recovered in a microtube, and then dried thoroughly using a centrifugal vacuum drier.

(ii) Fragmentation process of the labeled products

To each of the labeled products of the DNA derived from respective genomes in dry state obtained in the above i) of (4), a solution having a composition of 0.04 M Tris-acetate (pH 8.1), 0.1 M potassium acetate, and 0.03 M magnesium acetate tetrahydrate is prepared and added, and then mixed in suspension. The suspension is heat-treated at 94°C for about 15 minutes, and the labeled products of DNA fragments with 100 to 300 bases derived from respective genomes (Cy3-labeled product, Cy5-labeled product) are obtained.

The Cy3-labeled product and the Cy5-labeled product obtained are each placed onto an ultrafiltration column and centrifuged at 14,000 rpm for about 4 minutes, and each concentrated solution is recovered in a microtube, and then dried thoroughly using a centrifugal vacuum drier and the like.

Subsequently, to this microtube, a reagent solution which is prepared by combining salmon sperm DNA, formamide and ArrayHyb Hybridization buffer is added, and the dry material obtained above is mixed in suspension, and followed by incubation at 95°C for about 5 minutes. Thereby, a mixed solution of the Cy3Cy5-labeled products (a mixed solution of the fragmentation product of the Cy5-labeled product of the genomic DNA derived from *Chlamydophila caviae* and the fragmentation product of the Cy3-labeled product of the genomic DNA derived from *Chlamydia psttaci*) is prepared.

(5) Microarray hybridization (DNA-DNA hybridization on the array)

Next, for the microarray of Whole Genome Shotgun clone of genomic DNA derived from *Chlamydophila caviae,* hybridization with Cy3Cy5-labeled products is carried out by the conventional procedure.

For example, on a microarray of Whole Genome Shotgun clone of genomic DNA derived from *Chlamydophila caviae* obtained in the above-described step (3), a mixed solution of Cy3Cy5-labeled products prepared in the above-described (ii) of (4) is placed, and kept at 65°C under light shielding for not less than 8 hours to allow hybridization. After hybridization, the microarray is dipped in a 2 x SSC-0.1% SDS solution together with the cover glass at room temperature, and the cover glass is removed. After sequential washing with 1 x SSC solution containing 0.03% SDS (60°C) for 10 minutes, 0.2 x SSC solution (42°C) for 10 minutes and 0.05 x SSC solution (room temperature) for 10 minutes, the microarray is dried by centrifugation at 800 rpm for 5 minutes.

(6) Measurement of fluorescence intensity; from detection of signal to quantification

Using a fluorescence readout scanner, the fluorescence intensity from the microarray on which the microarray hybridization has been carried out as described in the above (5) is measured. On this occasion, the fluorescence intensity is measured by 2 channels of Cy3 and Cy5, and fluorescence detection data are obtained.

The Cy5-labeled product used for hybridization is a group of labeled DNA fragments prepared using the genomic DNA derived from *Chlamydophila caviae* as a material, and the Cy3-labeled product is a group of labeled DNA fragments prepared using genomic DNA for control as a material. Therefore, in the measurement of fluorescence intensity from Cy3 and Cy5 of a certain spot on a microarray, when the fluorescence intensity ratio of Cy5 for Cy3 is high, it indicates that the DNA fragment (PCR product) in the spot has hybridized more strongly with the Cy5-labeled product, namely, with the genomic DNA derived from *Chlamydophila caviae.* And the specificity of the DNA fragment (PCR product) for *Chlamydophila caviae* is deemed to be high.

On the other hand, in the measurement of fluorescence intensity from Cy3 and Cy5 of a certain spot, when the fluorescence intensity ratio of Cy5 for Cy3 is low, it indicates that the DNA fragment (PCR product) in the spot has hybridized with the Cy3-labeled product, namely with the genomic DNA for control. In this case, and the case when the fluorescence intensity from Cy3 and Cy5 are detected in the same level, or no fluorescence of both Cy3 and Cy5 is detected, the specificity of the DNA fragment (PCR product) for *Chlamydophila caviae* is deemed to be low.

And so, for example, on the basis of the fluorescence intensity ratio of Cy3/Cy5 (Ratio) detected on the microarray, the results are analyzed, for example, by making up a scatter chart (scatter plot). And screening for a specific sequence of *Chlamydophila caviae* is carried out.

As a result of screening, the spot (clone) which provides a signal specific for *Chlamydophila caviae* (when the fluorescence intensity from Cy5 is strong) is selected. The clone of this spot comprises the target oligonucleotide which hybridizes specifically with the nucleotide sequence of a *Chlamydophila caviae* gene.

Subsequently, using equipment usually used in this field such as a sequencer, and according to the conventional procedures, the nucleotide sequence of the obtained clone is determined, and thereby whether the target oligonucleotide has been obtained may be identified.

The primer for detection of *Chlamydophila caviae* involved in the present invention includes a primer comprising an oligonucleotide which comprises a part or the entire of a nucleotide sequence selected from SEQ ID NO:1, SEQ ID NO:2, SEQ ID NO:3, SEQ ID NO:4, SEQ ID NO:5 and SEQ ID NO:6, or a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6, and which is capable of hybridizing with the nucleotide sequence of a *Chlamydophila caviae* gene (hereinafter, optionally designates as primer of the present invention).

In addition, the primer of the present invention may be designed, in compliance with the condition of the nucleic acid amplification reaction such as PCR (including the real-time PCR), the condition of nucleic acid hybridization and the like, by selecting an appropriate region and an appropriate length in consideration of melting temperature (Tm value) and the like from oligonucleotide which comprises a part or the entire of a nucleotide sequence selected from SEQ ID NO:1, SEQ ID NO:2, SEQ ID NO:3, SEQ ID NO:4, SEQ ID NO:5 and SEQ ID NO:6, or a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6.

Preferably, the primer includes an oligonucleotide having a length with 10 to 50 nucleotides, more preferably 10 to 35 nucleotides, further more preferably 18 to 25 nucleotides which is considered to be a necessary number of nucleotide for retaining specificity as a primer.

As to a method for designing primer, the primer may be designed using software commonly used for designing primer such as, for example, a primer design tool on the web, Primer 3 (Whitehead Institute for Biomedical Research) and the like.

A specific example of an oligonucleotide to be used for the primer of the present invention (the oligonucleotide of the present invention), which comprises a part or the entire of a nucleotide sequence selected from SEQ ID NO:1, SEQ ID NO:2, SEQ ID NO:3, SEQ ID NO:4, SEQ ID NO:5 and SEQ ID NO:6, or a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6, and which is capable of hybridizing with the nucleotide sequence of a *Chlamydophila caviae* gene, is the same as described in the above explanation for the oligonucleotide of the present invention.

A specific example of the primer of the present invention includes, for example, the one which comprises an oligonucleotide comprising a part or the entire of a nucleotide sequence selected from SEQ ID NO:7 to 39, or a part or the entire of a sequence complementary to the nucleotide sequence selected from SEQ ID NO:7 to 39, and which is capable of hybridizing with the nucleotide sequence of a *Chlamydophila caviae* gene.

Preferable primer includes an oligonucleotide which comprises a part or the entire of a nucleotide sequence selected from SEQ ID NO:7 to 28, and which is capable of hybridizing with the nucleotide sequence of a *Chlamydophila caviae* gene, or an oligonucleotide which comprises a part or the entire of a sequence complementary to the nucleotide sequence selected from SEQ ID NO:7 to 28, and which is capable of hybridizing with the nucleotide sequence of a *Chlamydophila caviae* gene.

It should be noted that, the primers having a nucleotide sequence shown in SEQ ID NO:7 to 10 are designed based on the nucleotide sequence shown in SEQ ID NO: 1.

The primers having a nucleotide sequence shown in SEQ ID NO:11 to 14 are designed based on the nucleotide sequence shown in SEQ ID NO:2.

The primers having a nucleotide sequence shown in SEQ ID NO:15 to 18 are designed based on the nucleotide sequence shown in SEQ ID NO:3.
The primers having a nucleotide sequence shown in SEQ ID NO:19 to 22 are designed based on the nucleotide sequence shown in SEQ ID NO:4.
The primers having a nucleotide sequence shown in SEQ ID NO:23 to 26 are designed based on the nucleotide sequence shown in SEQ ID NO:5.
The primers having a nucleotide sequence shown in SEQ ID NO:27 to 28 are designed based on the nucleotide sequence shown in SEQ ID NO:6.
In addition, in the nucleotide sequence shown in SEQ ID NO:1, locations of the nucleotide sequences which have been designed as primers having nucleotide sequences shown in SEQ ID N0:7 to 10 are as follows, respectively:

SEQ ID NO:7 (R08_4f_Fw1): 145th to 163rd;
SEQ ID NO:8 (R08_4f_Rv1): 285th to 304th;
SEQ ID NO:9 (R08_4f_Fw2): 365th to 385th;
SEQ ID NO:10 (R08_4f_Rv2): 509th to 529th.

In the nucleotide sequence shown in SEQ ID NO:2, locations of the nucleotide sequences which have been designed as primers having nucleotide sequences shown in SEQ ID NO:11 to 14 are as follows, respectively:

SEQ ID NO:11 (R08_3d_Fw1): 37th to 56th;
SEQ ID NO:12 (R08_3d_Rv1): 154th to 171st;
SEQ ID NO:13 (R08_3d_Fw2): 575th to 594th;
SEQ ID NO:14 (R08_3d_Rv2): 743rd to 764th.

In the nucleotide sequence shown in SEQ ID NO:3, locations of the nucleotide sequences which have been designed as primers having nucleotide sequences shown in SEQ ID NO:15 to 18 are as follows, respectively:

SEQ ID NO:15 (R12_2a_Fw1): 627th to 645th;
SEQ ID NO:16 (R12_2a_Rv1): 787th to 806th;
SEQ ID NO:17 (R12_2a_Fw2): 96th to 117th;
SEQ ID NO:18 (R12_2a_Rv2): 213th to 235th.

In the nucleotide sequence shown in SEQ ID NO:4, locations of the nucleotide sequences which have been designed as primers having nucleotide sequences shown in SEQ ID NO:19 to 22 are as follows, respectively:

SEQ ID NO:19 (R12_4h_Fw1): 261st to 280th;
SEQ ID NO:20 (R12_4h_Rv1): 436th to 460th;
SEQ ID NO:21 (R12_4h_Fw2): 44th to 66th;
SEQ ID NO:22 (R12_4h_Rv2): 173rd to 192nd.

In the nucleotide sequence shown in SEQ ID NO:5, locations of the nucleotide sequences which have been designed as primers having nucleotide sequences shown in SEQ ID NO:23 to 26 are as follows, respectively:

SEQ ID NO:23 (R10_1g_Fw1): 48th to 68th;
SEQ ID NO: 24 (R10_1g_Rv1): 219th to 238th;
SEQ ID NO:25 (R10_1g_Fw2): 244th to 263rd;
SEQ ID NO:26 (R10_1g_Rv2): 424th to 443rd.

In the nucleotide sequence shown in SEQ ID NO:6, locations of the nucleotide sequences which have been designed as primers having nucleotide sequences shown in SEQ ID NO:27 to 28 are as follows, respectively:

SEQ ID NO:27 (R10_12d_Fw1): 4103rd to 4122nd;
SEQ ID NO:28 (R10_12d_Rv1): 4228th to 4248th.

It should be noted that, in the above description, the name of the primer denominated in the present invention is shown in parenthesis next to each SEQ ID NO.
**[0097]** The method for obtaining the primer of the present invention is as described above in the method for obtaining the nucleotide of the present invention.

In addition, the primer of the present invention may be labeled with a labeling substance.

The method for labeling the primer of the present invention includes the labeling methods of the oligonucleotide usually conducted in this field, and the methodology may be selected appropriately depending on the labeling substance.

As to the labeling substance to be used for labeling the primer of the present invention, any kind of the known labeling substances such as radioisotope and enzyme, fluorescent substance, luminescent substance, biotin and the like may be used.

For example, the radioisotope such as $^{32}P$, $^{33}P$, $^{35}S$; the enzyme such as alkaline phosphatase, horseradish peroxydase and the like; the fluorescent substance such as Alexa555, Alexa647 (manufactured by Invitrogen Corp.), Cyanine dye type of Cy3, Cy5 (manufactured by Amersham Biosciences K.K.), fluorescein and the like; the luminescent substance such as chemo luminescent reagents including Acridinium Ester and the like, are included.

The method for labeling the primer of the present invention with a radioisotope includes the method of labeling by incorporation of a radioisotope-labeled nucleotide into a primer at the time when the primer is synthesized, or labeling with a radioisotope after the primer is synthesized and the like. Specifically, commonly used random primer method, nick-translation method, 5'-terminal labeling method using T4 polynucleotide kinase, 3'-terminal labeling method using terminal deoxynucleotidyl transferase, RNA labeling method and the like are included.

The method for labeling the primer of the present invention with enzyme includes direct labeling methods of conventional technique in this field, in which an enzyme molecule such as alkaline phosphatase, horseradish peroxidase and the like is directly and covalently linked to the primer to be labeled.

The method for labeling the primer of the present invention with fluorescent substance includes, for example, a method in which the fluorescent-labeled nucleotide is incorporated into the primer by a conventional labeling technique in this field. In addition, also, by a method of replacing a nucleotide in the oligonucleotide sequence with a nucleotide having a linker arm (see, for example, Nucleic Acids Res., 1986, vol. 14, p. 6115), the nucleotide can be labeled with fluorescent substance. In this case, there may be a method in which a uridine having a linker arm on 5-position is synthesized chemically from deoxyuridine by a synthesis method disclosed in JP-A-60-500717, and an oligonucleotide which comprises the deoxyuridine is synthesized, subsequently a fluorescent substance is introduced into the oligonucleotide chain (JP-A-60-50717).

The method for labeling the primer of the present invention with a luminescent substance or with biotin includes the conventional technique of luminescent-labeling or biotin-labeling which is usually performed for nucleotides in this field.

The probe for detection of *Chlamydophila caviae* involved in the present invention includes a probe comprising an oligonucleotide (the oligonucleotide of the present invention) which comprises a part or the entire of a nucleotide sequence selected from SEQ ID NO:1, SEQ ID NO:2, SEQ ID NO:3, SEQ ID NO:4, SEQ ID NO:5 and SEQ ID NO:6, or a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6, and which is capable of hybridizing with the nucleotide sequence of a *Chlamydophila caviae* gene (hereinafter, sometimes designates as the probe of the present invention).

The probe of the present invention may be designed, in compliance with the condition of the nucleic acid amplification reaction such as PCR (including the real-time PCR) and the condition of nucleic acid hybridization and the like, and by selecting an appropriate region and an appropriate length in consideration of melting temperature (Tm value) and the like from oligonucleotide which comprises a part or the entire of a nucleotide sequence selected from SEQ ID NO:1, SEQ ID NO:2, SEQ ID NO:3, SEQ ID NO:4, SEQ ID NO:5 and SEQ ID NO:6, or a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6. In this regard, however, if the probe is intended to retaine sufficient specificity, it is desirable to design the probe in consideration of number of nucleotide necessary for retaining specificity as a probe.

For example, as to the probe to be used for the nucleic acid hybridization method (for example, Southern hybridization), it is preferable for the probe to have a length of 10 to 700 nucleotides, preferably 100 to 600 nucleotides, further preferably 200 to 500 nucleotides.

In addition, for example, as to the probe to be used for the real-time PCR amplification method (for example, TaqMan™ method, Molecular Beacon method, and so on), it is preferable for the probe to have a length of 10 to 50 nucleotides, preferably 15 to 40 nucleotides, further preferably 20 to 30 nucleotides.

A specific example of the oligonucleotide to be used for the probe of the present invention (the oligonucleotide of the present invention), which comprises a part or the entire of a nucleotide sequence selected from SEQ ID NO:1, SEQ ID NO:2, SEQ ID NO:3, SEQ ID NO:4, SEQ ID NO:5 and SEQ ID NO:6, or a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6, and which is capable of hybridizing with the nucleotide sequence of a *Chlamydophila caviae* gene, is the same as described in the above explanation for the oligonucleotide of the present invention.

A specific example of preferable probe of the present invention include, for example, a probe comprising an oligonucleotide which comprises a part or the entire of a nucleotide sequence selected from SEQ ID NO: 7 to 39, or an oligonucleotide which comprises a part or the entire of a sequence complementary to the nucleotide sequence selected from SEQ ID NO: 7 to 39, and which is capable of hybridizing with the nucleotide sequence of a *Chlamydophila caviae* gene.

A specific example of more preferable probe of the present invention includes an oligonucleotideoligonucleotide comprising an oligonucleotide which comprises a part or the entire of a nucleotide sequence selected from SEQ ID NO: 29 to 39, and which is capable of hybridizing with the nucleotide sequence of a *Chlamydophila caviae* gene.

It should be noted that, the nucleotide sequence selected from SEQ ID NO:29 to 39, or the complementary sequence thereto is a nucleotide sequence of an oligonucleotide to be amplified by the PCR using the primer of the present invention. The combination of the forward primer and the reverse primer, and the SEQ ID NO of the nucleotide sequence to be amplified by the PCR using such combination are shown collectively in Table 1. For example, it is indicated that the nucleotide sequence shown in SEQ ID NO:29 is a nucleotide sequence of the oligonucleotide to be amplified by the PCR using an oligonucleotide having a nucleotide sequence shown in SEQ ID NO:7 as a forward primer and an oligonucleotide having a nucleotide sequence shown in SEQ ID NO:8 as a reverse primer.

Table 1

| No | Forward primer | Reverse primer | Sequence to be amplified |
|---|---|---|---|
| 1 | SEQ ID NO:7 | SEQ ID NO:8 | SEQ ID NO:9 |
| 2 | 9 | 10 | 30 |
| 3 | 11 | 12 | 31 |
| 4 | 13 | 14 | 32 |
| 5 | 15 | 16 | 33 |
| 6 | 17 | 18 | 34 |
| 7 | 19 | 20 | 35 |
| 8 | 21 | 22 | 36 |
| 9 | 23 | 24 | 37 |
| 10 | 25 | 26 | 38 |
| 11 | 27 | 28 | 39 |

The method for obtaining the probe of the present invention is same as described above in the method for obtaining the nucleotide of the present invention.

The probe of the present invention may be labeled with a labeling substance.

As to the labeling substance to be used for labeling the probe of the present invention, any of the known labeling substances such as radioisotope and enzyme, fluorescent substance, luminescent substance, biotin and the like may be used.

A specific example of the labeling substance and the labeling method to be used for labeling the probe of the present invention include the same method as described in the labeling method of the primer of the present invention.

In addition, the labeled probe to be used in the detection method by the real-time PCR as described later includes the probe of the present invention which has been labeled with a labeling substance usually used in the real-time PCR method. For example, the labeled probe of the present invention in which the 5'-terminal has been labeled with a reporter fluorescent substance [carboxyfluorescein (FAM), hexachlorofluorescein (HEX), tetrachlorofluorescein (TET) and the like] and 3'-terminal has been labeled with a quencher dye [for example, a fluorescent substance such as carboxytetramethylrhodamine (TAMRA), nonfluorescent substance such as Black Hole Quencher dye (BHQ) and 4-((4-(dimethylamino) phenyl)azo)benzoic acid (DABCYL), and the like] is included.

In the method for detection by the TaqMan™ real-time PCR method to be described hereinafter, the above-described labeled probe can also be used.

Sample to be used for the detection of *Chlamydophila caviae* involved in the present invention includes various kinds of clinical specimen such as urine, urethral swab suspension, cervical swab suspension, oral swab suspension and so on. Before carrying out the detection process, these specimens may be subjected to a treatment, as a pretreatment in advance, such as concentration and separation of the bacteria which may exist in the specimen, and isolation and concentration of nucleic acid from the bacterial cell. Such method includes the treatment by enzyme, surface active agent, alkali, heat, etc. The sample may be the microbial cell isolated and cultured from a specimen; the nucleic acid isolated and purified from such microbial cell; or the nucleic acid amplified by the nucleic acid amplification detection system and the like.

The extraction and purification of the DNA from the above-described samples may be carried out according to the conventional procedures usually used for the extraction of Chlamydia DNA from a specimen.

For example, it may be carried out by the following method.
First, the cell wall of microbial cell in the sample is needed to be broken down. The method for this purpose includes, for example, in the case where the microbial cell is used as a sample, a method for disruption of the membrane structure of chlamydia by treating the microbial cell with protein denaturing agent, for example surface active agent such as SDS, guanidine thiocyanate (GTC) and the like, and a method of physical disruption of the microbial cell using glass beads and the like.

After disruption of the cell wall of the chlamydia, extraction and purification of DNA may be carried out by a common method for preparation of DNA in this field (phenol-chloroform extraction, ethanol precipitation method, the method described in Rapid and simple method for purification of nucleic acids, J. Clin. Microbiol., 1990, Mar;28 (3), 495-503, Boom R, Sol CJ, Salimans MM, Jansen CL, Wertheim-van Dillen PM, van der Noordaa J, or the method for precipitation using isopropanol and the like).

For the extraction and purification of DNA, various types of kits for this purpose are available on the market, and such kits may be utilized. For example, the extraction and purification of the DNA may be carried out using an ion-exchange resin type DNA extraction and purification kit Genomic-tip (manufactured by Quiagen GmbH) and the like.

The method for detection of *Chlamydophila caviae* involved in the present invention includes a method that utilizes an oligonucleotide (the oligonucleotide of the present invention) which comprises a part or the entire of a nucleotide sequence selected from SEQ ID NO:1, SEQ ID NO:2, SEQ ID NO:3, SEQ ID NO:4, SEQ ID NO:5 and SEQ ID NO:6, or a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6, and which is capable of hybridizing with the nucleotide sequence of a *Chlamydophila caviae* gene as a primer and/or a probe (the method using the primer and/or the probe of the present invention).

The above methods include, for example,

(A) A method in which, using the oligonucleotide of the present invention as a primer, the nucleic acid amplification reaction is carried out, then the obtained primer extension product is detected;
(B) A method in which the oligonucleotide of the present invention is labeled with a labeling substance, and the labeled oligonucleotide is used as a labeled probe; and the like. Each method will be described below.

(A) A method in which, using the oligonucleotide of the present invention as a primer, the nucleic acid amplification reaction is carried out, then the obtained primer extension product is detected
In the method (A), the method for performing the nucleic acid amplification reaction using the oligonucleotide of the present invention as a primer includes, for example, a method in which the primer extension is taken place by carrying out the nucleic acid amplification reaction by DNA polymerase using the primer of the present invention and using nucleic acid in a sample as a template [for example, the polymerase chain reaction (PCR) method; LAMP (Loop-mediated Isothermal Amplification) method (Tsugunori Notomi et al., Nucleic Acid Res., 28, e63, 2000), ICANTM (Isothermal and Chimeric primer-initiated Amplification of Nucleic acids) method (Rinsho Byori (Clinical Pathology), 51(11), 1061-1067, 2003, Nov), LCR (ligase chain reaction) method (JP-A-4-211399), SDA (strand displacement amplification) method (JP-A-8-19394)]. And, by this method, the sequence of a specific region of the nucleotide sequence of a *Chlamydophila caviae* gene can be amplified, and thus *Chlamydophila caviae* can be detected by measuring the obtained primer extension product.

Among the above-described methods for the nucleic acid amplification reaction, the PCR method is quoted as the most common method; and an example of the PCR method includes, for example, the real-time amplification detection method (see, for example, the description in USP-5210015 and USP-5538848). In addition, as an example of the detection method by the real-time amplification detection method, for example, the real-time PCR detection method is included.

An example of the real-time PCR detection method includes TaqMan™ real-time PCR method (see, for example, the description in USP-5538848), MGB Eclipse Probe System method (see, for example, the description in USP-5,801,155), Molecular Beacons Probe Technology method (see, for example, the description in USP-5925517), LUX Fluorogenic Primer method (Invitrogen Corp.), Quenching probe-PCR (QP) method (see, for example, the description in USP-6,492,121), and the like.

A specific example of the primer of the present invention to be used in the nucleic acid amplification reaction such as the PCR are as described above.

In addition, preferable combinations of the forward primer and the reverse primer to be used in the nucleic acid amplification reaction include the combinations shown in the above-described Table 1.

In Table 1, for example, the combination number 1 represents "the forward primer is an oligonucleotide which comprises a nucleotide sequence shown in SEQ ID NO:7; the reverse primer is an oligonucleotide which comprises a nucleotide sequence shown in SEQ ID NO:8".

Other reagents such as deoxyribonucleoside triphosphate (dATP, dCTP, dGTP, dTTP), DNA polymerase to be used for the nucleic acid amplification reaction such as the real-time PCR using the above-described primers may be the same reagents as used commonly in this field; and except for the use of the primer and the probe of the present invention, the condition and the procedures etc. may be performed according to general protocol of the PCR method.

The method for detection of the primer extension product obtained by the nucleic acid amplification reaction may be the conventional procedures commonly performed in this field, and is not limited specifically.

The detection method includes, various detection methods such as, for example, TaqMan™ real-time PCR method (see, for example, the description in USP-5538848); Intercalator method; MGB Eclipse Probe System method (see, for example, the description in USP-5,801,155); Molecular Beacons Probe Technology method (see, for example, the description in USP-5925517); LUX Fluorogenic Primer method (Invitrogen Corporation); Quenching probe-PCR (QP) method (see, for example, the description in USP-6,492,121); a method in which, after the nucleic acid amplification reaction is carried out, the primer extension products obtained are subjected to electrophoresis, and detection is performed based on the results; a method in which the nucleic acid amplification reaction is carried out using a labeled primer, and then the signal derived from the obtained primer extension product is measured; and the like.

Among them, the commonly used method includes, for example, the following methods:

(A-1) TaqMan™ real-time PCR method (TaqMan™ probe method);

(A-2) Intercalator method;
(A-3) The method in which, after the nucleic acid amplification reaction is carried out, the primer extension products obtained are subjected to electrophoresis, and the detection is performed based on the results of the electrophoresis; and
(A-4) The method in which the nucleic acid amplification reaction is carried out using a labeled primer, and then the signal derived from the obtained primer extension product is measured.

Each of these methods will be explained below.

(A-1) TaqMan™ real-time PCR method (TaqMan™ probe method)

This method is a real-time PCR method using a labeled probe which is labeled with a fluorescent dye (reporter) such as, for example, FAM on the 5'-terminal, and with a quencher dye such as, for example, TAMRA on the 3'-terminal, and is a method of detecting a small amount of target DNA with high sensitivity yet quantitatively (see, for example, the description in USP-5,538,848).

That is, the present method is a method in which, using the primer of the present invention, and a labeled probe which is labeled with a reporter fluorescent dye on the 5'-terminal and with a quencher dye on the 3'-terminal of the probe of the present invention, the PCR is carried out with the nucleic acid in a sample as a template, and then the signal derived from the labeling substance released from the labeled probe is detected.

The principle of the TaqMan™ real-time PCR method is as follows.

In this method, an oligonucleotide probe, which is labeled with a fluorescent dye (reporter) on the 5'-terminal thereof and with a quencher dye on the 3'-terminal thereof, and is capable of hybridizing with a specific region in the target gene, is used. In the probe, the fluorescence derived from the reporter is suppressed by the quencher dye under normal condition. Under a state where this fluorescent probe is hybridized completely with the target gene, the PCR is carried out from the outside thereof using a DNA polymerase. As the extension reaction by the DNA polymerase progresses, the fluorescent probe is hydrolyzed away from the 5'-terminal by the exonuclease activity of the DNA polymerase, and the released reporter dye generates the fluorescence. The real-time PCR method is a method for monitoring this fluorescence intensity in real time, and thereby, the initial amount of the template DNA can be quantified accurately.

In addition, the TaqMan™ real-time PCR detection method has very little generations of noise by nonspecific amplification reaction. Therefore, the method is a particularly excellent method in terms of the point that the amplification and detection of the target with higher specificity can be performed.

For the forward primer and the reverse primer to be used for the TaqMan™ real-time PCR detection method involved in the present invention, the primer of the present invention is utilized. The preferable primer includes the primer to be used in the nucleic acid amplification reaction such as the above-described PCR method, and the preferable combination thereof are also as described above.

The probe to be used for labeling with a fluorescent dye (reporter) on the 5'-terminal thereof and a quencher dye on the 3'-terminal thereof, and which is used for the TaqMan™ real-time PCR detection method involved in the present invention, may be the above-described probe of the present invention. In a practical sense, a probe comprising a nucleotide sequence of primer extension product which is anticipated to be obtained when the real-time PCR is carried out by the combined use of a selected forward primer and a reverse primer, or a probe comprising a nucleotide sequence designed further from such sequence may be used.

For example, the probe to be used when the real-time PCR is carried out using a primer R08_3d_Fw1 and a primer R08_3d_Rv1 includes an oligonucleotide comprising a part or the entire of a nucleotide sequence shown in SEQ ID NO:29 which is anticipated to be obtained when the real-time PCR is carried out.

The reporter fluorescent substance for labeling the 5'-terminal includes carboxyfluorescein (FAM), hexachlorofluorescein (HEX), tetrachlorofluorescein (TET), Cy5, VIC and the like, however, FAM is used commonly among them.

The quencher dye for labeling the 3'-terminal includes fluorescent substance such as carboxytetramethylrhodamine (TAMRA), nonfluorescent substance such as Black Hole Quencher dye (for example, BHQ2), 4-((4-(dimethylamino) phenyl)azo)benzoic acid (DABCYL), and TAMRA is used commonly among them.

Other reagents to be used for the real-time PCR detection method such as deoxyribonucleoside 3-phosphate (dATP, dCTP, dGTP, dTTP) and DNA polymerase may be the same reagents as usually used in the conventional real-time PCR, and the procedure of the real-time PCR may be carried out according to the customary protocol of the real-time PCR except for the use of the primer and the probe of the present invention.

As an example of the method for detection of *Chlamydophila caviae* by the TaqMan™ real-time PCR detection method involved in the present invention, taking a case where *Chlamydophila caviae* is detected using the "primer R08_3 d_Fw1" and the "primer R08_3d_Rv1" of the present invention as an example, the method would be explained as follows.

First, by a known method, purified DNA sample is obtained from a specimen (sample) to be detected for *Chlamydophila caviae.*

On the side, for example, using a DNA synthesizer, an oligonucleotide (R08_3d_Fw1) consisting of a nucleotide sequence shown in SEQ ID NO:11 and an oligonucleotide (R08_3d_Rv1) consisting of a nucleotide sequence shown in SEQ ID NO:12 are synthesized by the phosphoramidite method.

In addition, from a nucleotide sequence shown in SEQ ID NO:29 which is anticipated to be amplified by the PCR using a primer pair of R08_3d_Fw1 and R08_3d_Rv1, a sequence for use as a probe is designed, and an oligonucleotide having this nucleotide sequence is synthesized. The 5'-terminal of this oligonucleotide is coupled with a reporter dye of FAM, and the 3'-terminal with a reporter quencher of TAMRA by the conventional procedures, and thereby a fluorescence labeled probe is obtained.

Using the above-prepared R08_3d_Fw1 as a forward primer and the R08_3d_Rv1 as a reverse primer, the real-time PCR is carried out, for example, as follows.

That is, a 10 mM Tris-HCl buffer solution (pH 8.9) containing each 0.1 to 2 μM, preferably each 1 μM of the primer R08_3d_Fw1 and the primer R08_3d_Rv1, 100 to 1000 nM fluorescence-labeled probe, 1.0 to 4.0 mM $MgCl_2$, KCl, BSA, sodium cholate, 0.005 to 0.2% TritonX-100, each about 0.2 mM of dATP, dCTP, dGTP and dTTP, and 10 to 80 unit/mL of Taq DNA polymerase is prepared, and used as a reaction solution for PCR. To 20 μL of this reaction solution for PCR, 1 ng of the purified DNA sample is added, and obtained a sample for PCR.

Using this sample for PCR, and using appropriate real-time PCR detection equipment and the like, the real-time PCR is carried out. The reaction is repeated 30 to 50 cycles, and at every cycle, the fluorescence intensity derived from the reporter dye is measured.

In this instance, as for the method for detection of *Chlamydophila caviae,* when the fluorescence derived from the reporter dye is observed, it may be determined that *Chlamydophila caviae* exists (positive) in the sample.

In addition, in the real-time PCR method, a standard curve can be made up; and in consequence, the number of genomic DNA (copy number) of *Chlamydophila caviae* in the sample can be determined. Further, as the number is proportional to the number of *Chlamydophila caviae* cell, the number of *Chlamydophila caviae* in the sample can also be determined.

The method for preparing the standard curve may be performed according to the conventional procedure commonly carried out in the real-time PCR method. For example, using genomic DNA sample with known copy number derived from *Chlamydophila caviae* as a standard, a dilution series of concentration (copy number) of the DNA sample for PCR is prepared. After that, using each of the dilution series of the DNA sample for PCR, the real-time PCR is carried out according to the above-described method, and the fluorescence intensity derived from the reporter dye is measured. For each concentration of the dilution series of the DNA sample for PCR, the measured value of the fluorescence intensity (Rn, y-axis) is plotted for each cycle number of PCR (x-axis) to make up an amplification curve. After that, an Rn part where the fluorescence intensity is amplified exponentially is selected, and a threshold line (Th) is drawn. The crossing point of the Th with an amplification curve of each DNA sample for PCR is defined as threshold cycle (Ct). Subsequently, the Ct value (y-axis) is plotted for the logarithmic value of the copy number of each DNA sample used for PCR (x-axis), and an approximated curve obtained for each Ct may be used as a standard curve.

For the quantitative determination of the number of genomic DNA (copy number) of *Chlamydophila caviae* in the specimen, at first, the DNA is isolated and purified from the specimen to be detected for *Chlamydophila caviae,* and the real-time PCR of the obtained DNA sample is carried out, and an amplification curve is made up in the same manner. The Ct value at the point where the obtained amplification curve crosses the Th obtained when the standard curve is made, is obtained. By fitting the Ct value to the standard curve, the quantity (copy number) of genomic DNA of *Chlamydophila caviae* in the sample can be obtained.

(A-2) Intercalator method

Conventional intercalator method can be utilized, in which the real-time PCR is carried out using known intercalator.

For example, a method in which, using the primer of the present invention and the intercalator, the real-time PCR is carried out through the use of conventional intercalator method, is included.

That is, the intercalator is a reagent capable of generating fluorescence by binding specifically to the double-stranded DNA, and generates fluorescence when excitation light is irradiated. When the DNA is increased as the result of repeated amplification by the PCR, the intercalator is incorporated into the DNA accordingly. Because the amount of intercalator incorporated into the DNA will increase in proportion to the amount of amplification product generated, the amount of primer extension product can be determined by detecting the fluorescence intensity originated from the intercalator.

In this regard, however, because the intercalator binds to the entire double-stranded DNA, melting curve analysis may be carried out, as need arise, by drawing melting curve based on the measurement results of fluorescence intensity. Namely, after carrying out the PCR, the temperature of the reaction solution of PCR is increased gradually, the fluorescence intensity derived from the intercalator is measured simultaneously. In the beginning, the PCR amplification product generates fluorescence because it takes double stranded form. However, when temperature of the reaction solution of PCR reaches to a certain temperature, the amplification products will dissociate to single strand form, and therefore, the fluorescence intensity derived from the intercalator decreases rapidly. The temperature at this occasion is melting temperature (Tm value), and is an intrinsic value for the sequence of primer extension product. Whether the peak of melting curve corresponds to the peak of an objective specific product or a non-specific product can be determined from this Tm value.

In this intercalator method, any electrophoretic procedure after the real-time PCR is not necessary, and therefore, this is an effective method in the field of clinical testing and the like where a rapid determination is required.

As to the intercalator to be used in the present invention, any type of intercalator usually used in this field, for example, SYBR™ Green I (Molecular Probes Inc.), ethidium bromide, fluorine and the like can be utilized.

An example of "the method for detection of *Chlamydophila caviae* through the use of intercalator method" involved in the present invention will be explained as follows:

Using the primer of the present invention and the intercalator (for example, SYBR™ Green I), and using a purified DNA sample isolated from a specimen (sample) to be detected for *Chlamydophila caviae* as a template, the real-time PCR is carried out with the use of a polymerase such as Taq DNA polymerase. And, the fluorescence intensity derived from the intercalator which intercalates with the primer extension products in correlation with the amplified amount is measured.

Subsequently, by plotting the melting temperature of the primer extension product (double-stranded DNA) as horizontal axis and the first derivation (variation) of fluorescence intensity as vertical axis, melting curve is made. On the other side, the same measurment as descrived above except using a *Chlamydophila caviae* type strain is carried out, and then the detection of peak is examined. Using this, melting curve analysis of the primer extension product is carried out, and thereby detection of peak is examined. When a single peak is obtained for the sample, and the peak position is identical to that obtained using a type strain of *Chlamydophila caviae,* it can be determined that the sample is positive for *Chlamydophila caviae* (that is, there exists *Chlamydophila caviae* or the gene thereof; and hereinafter, the same as above).

Or otherwise, a dilution series of the purified DNA sample solution is prepared, and for each dilution series, the real-time PCR is carried out in the same way as described above.

In addition, based on the measurement value obtained by the method through the use of the intercalator method, a standard curve can also be made up according to the conventional procedure usually performed in the real-time PCR, and thereby, using the standard curve, the quantity (copy number) of genomic DNA of *Chlamydophila caviae* in a sample can be determined.

For example, using genomic DNA sample with known copy number derived from *Chlamydophila caviae* as a standard, a dilution series of concentration (copy number) of the DNA sample for PCR is prepared. After that, using each of the dilution series of the DNA sample for PCR, the real-time PCR is carried out according to the above-described method, and the fluorescence intensity derived from the intercalator is measured. For each concentration of the dilution series of the DNA sample for PCR, the measured value of the fluorescence intensity (Rn, y-axis) is plotted for each cycle number of PCR (x-axis) to make up an amplification curve. After that, the Ct value is obtained by the same procedures as described above. And, the Ct value (y-axis) is plotted for the logarithmic value of the copy number of each DNA sample used for PCR (x-axis), and an approximated curve obtained for each Ct may be used as a standard curve.

For the quantitative determination of the number of the genomic DNA (copy number) of *Chlamydophila caviae* in the specimen, at first, the DNA is isolated and purified from the specimen to be detected for *Chlamydophila caviae,* and the real-time PCR by the intercalator method for the obtained DNA sample is carried out, and an amplification curve is made up in the same manner. The Ct value at the point where the obtained amplification curve crosses the Th obtained when the standard curve is made, is obtained. By fitting the Ct value to the standard curve, the quantity (copy number) of genomic DNA of *Chlamydophila caviae* in the sample can be obtained.

As an example of the method for detection of *Chlamydophila caviae* by the real-time PCR detection method using the intercalator involved in the present invention, taking a case where *Chlamydophila caviae* is detected using the above-described "primer R08_3d_Fw1" and "primer R08_3d_Rv1" of the present invention, the method will be explained as follows.

At first, by known method, the purified DNA sample is obtained from a specimen (sample) for detection of *Chlamydophila caviae.*

On the side, for example, using a DNA synthesizer, an oligonucleotide (R08_3d_Fw1) consisting of the nucleotide sequence shown in SEQ ID NO:11 and an oligonucleotide (R08_3d_Rv1) consisting of the nucleotide sequence shown in SEQ ID NO:12 are synthesized by the phosphoramidite method.

Using the synthesized R08_3d_Fw1 as a forward primer and the R08_3d_Rv1 as a reverse primer, the real-time PCR is carried out, for example, as follows.

That is, a 10 mM Tris-HCl buffer solution (pH 8.9) containing each 50 to 2000 nM of the primer R08_3d_Fw1 and the primer R08_3d_Rv1, about 5 to 100000 times dilution of the concentrate solution of intercalator [for example, SYBR™ Green I (product name of Molecular Probe Inc.)], 1.0 to 4.0 mM $MgCl_2$, KCl, BSA, sodium cholate, 0.005 to 0.2% TritonX-100, each about 0.2 mM of dATP, dCTP, dGTP and dTTP, and 10 to 80 U/mL of polymerase (for example, Taq DNA polymerase) is prepared, and used as a reaction solution for PCR. To the reaction solution for PCR, the purified DNA sample purified from a specimen (sample) for detection of *Chlamydophila caviae* is added, and used as a sample for PCR. Using this sample for PCR, the real-time PCR is carried out using real-time PCR detection equipment and the like. The reaction is repeated 30 to 50 cycles, and at every cycle, the fluorescence intensity derived from the SYBR™ Green I which intercalates in correlation with the amplification quantity of the primer extension products is measured.

Subsequently, the melting curve is depicted by plotting the melting temperature of the primer extension product (double-stranded DNA) as horizontal axis and the first derivation (variation) of fluorescence intensity as vertical axis. Using the melting curve, the melting curve analysis of the primer extension product is carried out to detect the peak. When the obtained peak is a single peak, yet when having appeared in the same position as the position of the peak obtained by measuring similarly using a type strain of *Chlamydophila caviae,* it may be determined that the sample is positive for *Chlamydophila caviae.*

Further, by making a standard curve, number of the genomic DNA (the copy number) of *Chlamydophila caviae* in the sample can be obtained. In addition, as the number is proportional to number of *Chlamydophila caviae* cell, the number of *Chlamydophila caviae* in the specimen (sample) can also be determined.

(A-3) The method in which, after the nucleic acid amplification reaction is carried out, the primer extension products obtained are subjected to electrophoresis, and the detection is performed based on the results of the electrophoresis.
This method includes, for example, "the method for detection of *Chlamydophila caviae* characterized by comprising the following steps:

(i) the nucleic acid amplification reaction is carried out using an oligonucleotide which comprises a part or the entire of a nucleotide sequence selected from SEQ ID NO:1, SEQ ID NO:2, SEQ ID NO:3, SEQ ID NO:4, SEQ ID NO:5 and SEQ ID NO:6, or a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6, and which is capable of hybridizing with the nucleotide sequence of a *Chlamydophila caviae* gene as a primer (the primer of the present invention), and using the nucleic acid in a sample as a template, and
(ii) the primer extension product obtained in the above (i) is subjected to electrophoresis, and based on the results, the presence of *Chlamydophila caviae* is determined (detected)".

Specific examples of the nucleic acid amplification reaction are as described above.

The method for determination of the presence of *Chlamydophila caviae* based on the results of electrophoresis includes, for example,

(A-3-1) a method in which the determination is made by confirming a fraction of primer extension product having objective size (number of base pair); and
(A-3-2) a method in which the determination is made by hybridization using labeled probe.

Conditions, operation procedures and the like of the electrophoresis may be performed according to the conventional method usually carried out in this field.

The methods of (A-3-1) and (A-3-2) will be described below.

(A-3-1) The method in which the determination is made by confirming a fraction of primer extension product having objective size (number of base pair) For example, first, an appropriate combination of the forward primer and the reverse primer is selected from the primer of the present invention, and using it, the nucleic acid amplification reaction such as PCR is carried out.

Subsequently, the primer extension product obtained is subjected to the electrophoresis. From the combination of the forward primer and the reverse primer used for the nucleic acid amplification reaction, size (number of base pair) of the primer extension product which is anticipated to be amplified by the PCR is estimated in advance. And, whether the electrophoretic fraction obtained is relevant to the estimated size of amplification product may be confirmed by the conventional method. For example, a method in which, by such a way that the nucleic acid species is visualized by staining the obtained electrophoretic fraction with ethidium bromide and the like, the primer extension product is identified based on its characteristic size, is included.

Specific example of the method for determination by the method of (A-3-1) includes, for example, a method in which, after carrying out the PCR using a combination of the forward primer and the reverse primer listed in the above-described Table 1, the primer extension product obtained is subjected to electrophoresis, and when an oligonucleotide having the nucleotide sequence shown in SEQ ID NO described in Table 1, which is anticipated to be amplified by the combination of the primers, or a fraction having a size corresponding to the number of the base pair is identified, it may be determined that the sample is positive for *Chlamydophila caviae.*

Specific examples of the method of (A-3-1) will be shown collectively in the following Table 2.

That is, for example, the method of No. 1 in Table 2 is "a method in which, after carrying out the PCR using an oligonucleotide comprising a nucleotide sequence shown in SEQ ID NO:7 as a forward primer, and using an oligonucleotide comprising a nucleotide sequence shown in SEQ ID NO:8 as a reverse primer, the primer extension product obtained is subjected to electrophoresis, and when a fraction of 160 base pairs or a fraction of oligonucleotide having the nucleotide sequence shown in SEQ ID NO:29 is confirmed, the sample is determined to be positive".

Table 2

| No | Forward primer | Reverse primer | Detection target | |
|---|---|---|---|---|
| | | | Size of amplicon (bp) | Nucleotide sequence |
| 1 | SEQ ID NO:7 | SEQ ID NO:8 | 160 | SEQ ID NO:29 |
| 2 | 9 | 10 | 165 | 30 |
| 3 | 11 | 12 | 135 | 31 |
| 4 | 13 | 14 | 190 | 32 |
| 5 | 15 | 16 | 180 | 33 |
| 6 | 17 | 18 | 140 | 34 |
| 7 | 19 | 20 | 200 | 35 |
| 8 | 21 | 22 | 149 | 36 |
| 9 | 23 | 24 | 191 | 37 |
| 10 | 25 | 26 | 200 | 38 |
| 11 | 27 | 28 | 146 | 39 |

(A-3-2) The method in which the detection is made by hybridization using labeled probe
The method includes, for example, a method in which the primer extension product obtained by the nucleic acid amplification reaction is subjected to electrophoresis, and then the electrophoretic fraction obtained is tested for hybridization with a labeled probe which is the probe of the present invention labeled with a labeling substance. When the presence of a fraction hybridizing with the labeled probe is confirmed by detecting a signal derived from the labeled probe, it may be determined that the sample is positive for *Chlamydophila caviae.*

Specific examples of the probe to be used and the labeling substance for use in labeling the probe, and the method for labeling the probe are as described above.

An example would be described as follows.

That is, a method in which, after carrying out the PCR using a combination of the forward primer and the reverse primer listed in the above-described Table 1, the primer extension product obtained is subjected to electrophoresis. On the side, a labeled probe is prepared in advance by labeling an oligonucleotide with labeling substance, which comprises a part or the entire of a nucleotide sequence (the nucleotide sequence of "Detection target" in Table 2) anticipated to be amplified by the combinational use of the forward primer and the reverse primer in the PCR. The electrophoretic fraction is tested for hybridization with labeled probe, and when the presence of a fraction hybridizing with labeled probe is confirmed by detecting the signal derived from the labeled probe, it may be determined that the sample is positive for *Chlamydophila caviae,* is included.

The specific examples of these preferable methods are shown collectively in the following Table 3.

For example, the method of No. 1 in Table 3 is "a method in which, after carrying out the PCR using an oligonucleootide comprising a nucleotide sequence shown in SEQ ID NO:7 as a forward primer, and using an oligonucleootide comprising a nucleotide sequence shown in SEQ ID NO:8 as a reverse primer, the primer extension product obtained is subjected to electrophoresis. After that, the fraction obtained is tested for hybridization with a labeled probe prepared by labeling an oligonucleotide which comprises a nucleotide sequence comprising a part or the entire of a sequence shown in SEQ ID NO:29, and when the presence of a fraction hybridizing with labeled probe is confirmed by detecting a signal derived from the labeled probe, it is determined that the sample is positive".

Table 3

| No | Forward primer | Reverse primer | Probe |
|---|---|---|---|
| 1 | SEQ ID NO:7 | SEQ ID NO:8 | SEQ ID NO:29 |
| 2 | 9 | 10 | 30 |
| 3 | 11 | 12 | 31 |
| 4 | 13 | 14 | 32 |
| 5 | 15 | 16 | 33 |
| 6 | 17 | 18 | 34 |
| 7 | 19 | 20 | 35 |
| 8 | 21 | 22 | 36 |
| 9 | 23 | 24 | 37 |
| 10 | 25 | 26 | 38 |
| 11 | 27 | 28 | 39 |

The details of the method for detection of *Chlamydophila caviae* of the present invention by the method of (A-3) will be explained by taking a case as an example, where after the PCR is carried out using R08_3d_Fw1 (SEQ ID NO:11) as a forward primer and R08_3d_Rv1 (SEQ ID NO:12) as a reverse primer, and followed by electrophoresis, the detection is made by the method of identifying a fraction of the primer extension product having the objective number of base pair (the method of the above-described (A-3-1); Table 2, No. 3), as follows.

First, by a known method, purified DNA sample is obtained from a specimen (sample) to be detected for the presence of *Chlamydophila caviae.*

On the side, using a DNA synthesizer, R08_3d_Fw1 (an oligonucleotide consisting of a nucleotide sequence shown in SEQ ID NO:11) and R08_3d_Rv1 (an oligonucleotide consisting of a nucleotide sequence shown in SEQ ID NO:12) are synthesized by the phosphoramidite method.

Using the primer R08_3d_Fw1 and the primer R08_3d_Rv1, the PCR is carried out. The obtained reaction solution after PCR is subjected to 1.5% agarose gel electrophoresis. Subsequently, after staining the gel with ethidium bromide, the fluorescence generated by UV ray irradiation is detected. In addition, the molecular weight marker is also electrophoresed in the same time in parallel with the reaction solution, and a length of the detected DNA fragment is calculated by comparing the relative mobility. In the PCR using the R08_3d_Fw1 as a forward primer and the R08_3d_Rv1 as a reverse primer, it is anticipated that the DNA fragment with 135 base pair (having a nucleotide sequence shown in SEQ ID NO:31) in the nucleotide sequence of *Chlamydophila caviae* could be replicated (see Table 2, No. 3). Consequently, when a fluorescent band with the size of 135 base pair is confirmed, it may be determined that the sample is positive for *Chlamydophila caviae.*

In addition, in the nucleic acid amplification step of the present invention, a detection method through the use of RNA transcription product can be applied. For example, NASBA (nucleic acid sequence based amplification) method (JP-2650159), 3SR (self-sustained sequence replication) method (JP-B-7-114718), TAS (transcription based amplification system) method (JP-A-2-500565: WO 88/10315), TMA (transcription mediated amplification) method (JP-A-11-46778) and the like are included. Among them, the constant temperature nucleic acid amplification methods utilizing a concerted mode of action of reverse transcriptase and RNA polymerase (reaction is carried out under such condition that allows the reverse transcriptase and the RNA polymerase act as concertedly) is a suitable method when the determination system is intended to be automated.

(A-4) The method in which the nucleic acid amplification reaction is carried out using a labeled primer and the signal derived from the obtained primer extension product is measured.
The method of (A-4) includes a method in which, using a labeled primer prepared by labeling the primer of the present invention by the above-described method, and using nucleic acid in a sample as a template, the nucleic acid amplification reaction such as PCR is carried out; and the detection/measurement of signal derived from the obtained primer extension product is carried out; and when the signal is detected, it may be determined that the test sample is positive for *Chlamydophila caviae.*

The forward primer and the reverse primer to be used in this method include the ones which are used in the above-described PCR method, and the specific examples of preferable primers and preferable combination are also as described above.

In the above-described method, after the nucleic acid amplification reaction is carried out, free labeled primer is removed; and the signal derived from the primer extension product is measured; and when the signal is detected, it may be determined that the sample is positive for *Chlamydophila caviae.*

The method for removing free labeled primer includes a method in which, after the primer extension product in reaction mixture obtained by the nucleic acid amplification reaction is precipitated by conventional procedure of precipitating nucleic acid (ethanol precipitation method, a precipitation method using isopropanol and the like), the supernatant solution which contains non-precipitated free labeled primer is removed, and the like.

In addition, a method in which a reaction mixture obtained by nucleic acid amplification reaction is treated by a gel chromatography under appropriate condition, and thereby the primer extension product is separated from the free labeled primer, a method of separation by electrophoresis and the like are also included.

(B) A method in which the oligonucleotide of the present invention is labeled with a labeling substance, and used it as a labeled probe
Further, the method for detection of *Chlamydophila caviae* of the present invention includes a method in which, the oligonucleotide of the present invention is labeled with a labeling substance and used it as a labeled probe, and the labeled probe is allowed to hybridization with the nucleic acid in the sample, and after removing the free labeled probe, the signal derived from the hybridized complex is detected.

Specifically, for example, the following methods are included.

(B-1) A detection method in which, using an oligonucleotide of the present invention which has been bound to a solid carrier as a trapping probe, the hybridization with nucleic acid in the sample is carried out, and thereby the nucleic acid derived from *Chlamydophila caviae* is immobilized on the solid phase (see, for example, the description in JP-A-62-265999).

In the case of this method, the oligonucleotide of the present invention or the solid phase carrier may be labeled with a labeling substance.

(B-2) A method for carrying out a sandwich assay in which, using an unlabeled trapping probe (B-1) and the labeled probe which is the labeled probe of the present invention, the hybridization with nucleic acid in the sample is carried out to form a complex of trapping probe and nucleic acid derived from *Chlamydophila caviae* and labeled probe on the solid carrier, and then the signal derived from the labeled probe is measured (see, for example, the description in JP-A-58-40099).

(B-3) A method in which, using a biotin-labeled probe of the present invention, the hybridization with nucleic acid in the sample is carried out, and after that, the nucleic acid derived from *Chlamydophila caviae* in the sample is trapped by an avidin-bound carrier.

It should be noted that, as to the reagents to be used for the method for detection of *Chlamydophila caviae* of the present invention, any reagent usually used in this field, for example, buffering agent, stabilizer, preservatives and the like can be used, so long as such reagents do not inhibit the stability of the coexisting reagents nor inhibit the nucleic acid amplification reaction such as PCR and hybridization reaction. And, concentration of the reagent may be selected as appropriate from the range of concentration usually used in this field.

Specific example of buffer solution includes all kinds of buffer solutions usually used for performing PCR and hybridization reaction, for example, Tris buffer solution, phosphate buffer solution, Veronal buffer solution, borate buffer solution, Good’s buffer solution and the like; and the pH of the buffer solution is not particularly limited, but generally a range between pH 5 to pH 9 is preferable.

In addition, if necessary, nucleic acid synthetase (DNA polymerase, RNA polymerase, reverse transcriptase and the like), enzyme-specific substrate (dNTP, rNTP and the like), and additionally, double strand intercalator (ethidium bromide, SYBR™ Green and the like), and alternatively, substance for signal detection such as FAM and TAMRA may be used.

The reagent kit for detection of *Chlamydophila caviae* involved in the present invention includes "a reagent kit for detection of *Chlamydophila caviae* comprising an oligonucleotide as a primer (the primer of the present invention) and/or a probe (the probe of the present invention) which comprises a part or the entire of a nucleotide sequence selected from SEQ ID NO:1, SEQ ID NO:2, SEQ ID NO:3, SEQ ID NO:4, SEQ ID NO:5 and SEQ ID NO:6, or a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6, and which is capable of hybridizing with the nucleotide sequence of a *Chlamydophila caviae* gene".

Specific examples of the primer of the present invention and the probe of the present invention which constitute the above-described kit are as described hereinbefore in the explanation for the "the primer of the present invention" and "the probe of the present invention".

The primer of the present invention may be the one which is labeled with a labeling substance. Specific example of the labeling substance is as described above.

The kit comprising the primer of the present invention also encompasses a composition comprising a pair of the forward primer and the reverse primer. A preferable combination of the primer pair is as described hereinbefore.

In addition, the above-described kit may further comprise the oligonucleotide of the present invention which has been labeled with a labeling substance as a labeled probe.

Further, the kit of the present invention includes, "a reagent kit for detection of *Chlamydophila caviae* comprising an oligonucleotide (the oligonucleotide of the present invention) as a probe which comprises a part or the entire of a nucleotide sequence selected from SEQ ID NO:1, SEQ ID NO:2, SEQ ID NO:3, SEQ ID NO:4, SEQ ID NO:5 and SEQ ID NO:6, or a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6, and which is capable of hybridizing with the nucleotide sequence of a *Chlamydophila caviae* gene".

The probe may be a one which is labeled with a labeling substance.

Preferable aspect and specific examples of constituent reagents which configure these kits are as described above.

It should be noted that, the reagent kit for detection of *Chlamydophila caviae* of the present invention may comprise, for example, buffering agent, stabilizer, preservatives and the like which neither inhibit the stability of the coexisting reagents and the like nor inhibit the nucleic acid amplification reaction such as PCR and the hybridization reaction. In addition, the concentrations of the reagents may be selected as appropriate from the range of concentration usually used in this field.

Specific example of buffer solution includes all kinds of buffer solutions usually used for performing the PCR and the hybridization reaction, for example, Tris buffer solution, phosphate buffer solution, Veronal buffer solution, borate buffer solution, Good's buffer solution and the like, and the pH is not particularly limited, but generally a range between pH 5 to pH 9 is preferable.

In addition, if necessary, the nucleic acid synthetase (DNA polymerase, RNA polymerase, reverse transcriptase and the like), the substrate relevant to the enzyme (dNTP, rNTP and the like), and additionally, the double strand intercalator (SYBR™ Green, ethidium bromide and the like), and alternatively, the substance for signal detection such as FAM and TAMRA, may be comprised in the kit.

Hereinafter, the present invention will be further explained in detail by referring to the following Examples, but the scope of the present invention should not be limited thereto.

It should be noted that, all bacteria used in Examples are clinical isolates, and their bacterial species has already been differentiated after culturing by colony morphology and conventional various types of biochemical tests on the cultured bacterium.

## EXAMPLE

Example 1: Selection of a clone derived from genomic DNA of *Chlamydophila caviae*

(1) Preparation of DNA sample derived from *Chlamydophila caviae*

*Chlamydophila caviae* (Okayama University SC10 strain) was granted by Professor Hiromi Kumon of School of Medicine, Okayama University. From *Chlamydophila caviae* (Okayama University SC10 strain) which was cultured by a conventional method, purified genomic DNA was obtained by a conventional method (hereinafter, optionally referred to as "purified genomic DNA derived from *Chlamydophila caviae*"). The obtained genomic DNA was adjusted to 400 ng/

μL final concentration (in 10 mM Tris-HCl buffer solution, pH 8.9), and used it as a "DNA sample derived from *Chlamydophila caviae".*

(2) Preparation of Whole Genome Shotgun Library

Using a 24 μg of the DNA sample derived from *Chlamydophila caviae* obtained in (1) above as a material, the Whole Genome Shotgun Library was prepared by the following method (a modified method from Whole Genome Shotgun method described in Science 2001 Feb 16; 291 (5507): 1304-1351 Venter et al.).

First, the DNA sample derived from *Chlamydophila caviae* was fragmented by treatment using a nebulizer (manufactured by Invitrogen Corp.) in the presence of 20% final concentration of glycerol under the pressure of 5 kPa to 9 kPa for about 10 minutes. By this treatment, a fraction (DNA fragment) with the objective size of 500 to 1,000 bp was recovered efficiently. The fraction obtained was purified using an extraction column manufactured by Quiagen GmbH.

Subsequently, using the DNA Blunting Kit (manufactured by Takara Bio Inc.) and through the use of 5' → 3' polymerase activity and 3' → 5' exonuclease activity of T4 DNA Polymerase, terminal of the obtained DNA fragment was blunted. This blunt-ended DNA fragment was subjected to ligation reaction with blunt-ended pBSII $sk^+$ vector (manufactured by Stratagene Corp.), and a recombinant DNA of pBSII $sk^+$ vector (*amp*$^r$), into which the DNA fragment had been inserted, was prepared.

Using *E. coli* JM109 Competent Cells (manufactured by Takara Bio Inc.), transformation of the *E. coli* JM109 Competent Cells was carried out using the above obtained recombinant DNA according to a protocol of the product.

The transformant obtained was cultured in a LB-agarose medium containing 100 μg/mL ampicillin, 0.2 mM IPTG and 40 μg/mL X-Gal. White colonies were picked up, and thus a library of the transformant in which "the recombinant DNA incorporated with the objective DNA fragment" has been integrated (Whole Genome Shotgun clone Library derived from genomic DNA of *Chlamydophila caviae*) was obtained.

(3) Preparation of microarray

Using the library of the transformant obtained in (2) above (Whole Genome Shotgun clone Library of the genomic DNA derived from *Chlamydophila caviae),* the PCR was carried out by the following method, and a probe material to be fixed on a slide glass was prepared.

Firstly, a 10 mM Tris-HCl buffer solution (pH 8.9) containing 1 μM each of primer M13 Primer M1 (manufactured by Takara Bio Inc.) and primer M13 Primer RV (manufactured by Takara Bio Inc.), 1.5 mM $MgCl_2$, 80 mM KCl, 500 μg/mL BSA, 0.1% sodium cholate, 0.1% Triton X-100 (polyoxyethylene octylphenyl ether, product name of Rohm and Haas Co.), 0.2 mM each of dATP, dCTP, dGTP and dTTP, and 40 unit/mL of Taq DNA polymerase (manufactured by Nippon Gene Co. Ltd.) was prepared and used as a reaction solution for PCR.

The DNA was purified from each transformant (Whole Genome Shotgun clone of genomic DNA derived from *Chlamydophila caviae)* obtained in (2) above according to the conventional procedure. This purified DNA (which would be used as a template on performing PCR later) was added to 20 μL of the reaction solution for PCR and suspended, and the suspension prepared was used as a sample for PCR. Using this sample for PCR, 30 cycles of the PCR was carried out under the following reaction conditions using the DNA Thermal Cycler (DNA Engine PTC200; manufactured by MJ Research Inc.).

Reaction conditions of the PCR:

Heat denaturation: 94°C for 0.5 minutes;
Annealing: 55°C for 1 minute;
Polymerization reaction: 75°C for 0.5 minutes.

The PCR amplification product obtained was purified, and then mixed with immobilization buffer (final concentration: 3 x SSC).

The final concentration of the PCR amplification product to be spotted was adjusted to give 300 ng/μL, and using a typing instrument (GTMAS Stamp II; manufactured by Nippon Laser & Electronics Co.) which was set at 55% in humidity inside of the instrument, the PCR product obtained above was spotted (the spot diameter: 150 μm to 250 μm) on a slide glass (CMT GAPS-II; manufactured by Coming Inc.). The spotting-completed slide glass was transferred to a UV cross-linker (UV Stratalinker 1800; manufactured by Stratagene Corp.), and UV light of 150 mJ/$cm^2$ was irradiated to fix the PCR amplification product (the objective DNA) on the slide glass, and thus the microarray (a microarray made from the Whole Genome Shotgun clone Library of genomic DNA derived from *Chlamydophila caviae,* 2900 clones in total) was prepared.

(4) Fluorescent dye labeling of the target genomic DNA

(i) Fluorescent dye labeling of the target genomic DNA

Fluorescent dye labeling of the target genomic DNA was carried out using BioPrime DNA labeling system (manufactured by Invitrogen Corp.).

Firstly, after a 2 μg of purified genomic DNA derived from *Chlamydophila caviae* obtained in above (1) was mixed with 20 μL of random primer solution contained in the product of the labeling system kit, the mixture was subjected to heat denaturation treatment (95°C for 5 minutes), and thus the sample solution was used. On the side, the genomic DNA was extracted and purified from *Chlamydia psttaci* (cal-10 strain) by conventional procedure (genomic DNA for control); and the same treatment was carried out for the sample; and thus sample solution was obtained.

Subsequently, to each sample solution obtained, 2 μL of 0.1 M DTT, 2 μL of the mixed solution of dATP/dCTP/dGTP (each 5 mM), 0.8 μL of 2.5 mM dTTP, 1.6 μL of 5 mM Ha-dUTP and 1 μL of Klenow enzyme (40 U/μL) were added and adjusted to give the total volume 50 μL with sterile deionized water, and then the extension reaction was carried out at 37°C for 3 hours. An ultrafiltration column Microcon YM-30 (manufactured by Millipore Co.) was set to the attached 1.5 mL tube and the above obtained reaction product was placed on the column and centrifuged at 14,000 rpm for 4 minutes. The concentrated solution was recovered in a microtube and dried thoroughly using a centrifugal vacuum drier (CentriVap concentrator; manufactured by Labconco Co.).

The dried reaction product obtained above was added with 10 μL of 50 mM $NaHCO_3$ and mixed, then left standing at ambient temperature for 2 to 3 minutes (hereinafter referred to as "solution of reaction product").

Separately, 1 mg of Cy5 (manufactured by Amersham Biosciences K.K.) or Cy3 (manufactured by Amersham Biosciences K.K.) was dissolved in 105 μL of DMSO (Cy-dye Solution Cy3, Cy-dye Solution Cy5). A 10 μL aliquot of the Cy-dye Solution Cy5 was added to the above-described solution of reaction product which was obtained by the use of genomic DNA fragment derived from *Chlamydophila caviae,* and incubated (under light shielding) at 40°C for 60 minutes. Also, a 10 μl aliquot of the Cy-dye Solution Cy3 was added to the above-described solution of reaction product which was obtained by the use of genomic DNA for control (derived from *Chlamydia psttaci),* and also incubated (under light shielding) at 40°C for 60 minutes.

Further, to the above-described each reaction product of post incubation, a 10 μL of 4 M $NH_2OH$ (prepared just before use) was added and mixed, and was incubated (under light shielding) for 15 minutes to obtain the respective labeled product, namely, the labeled product of the Cy5-labeled genomic DNA derived from *Chlamydophila caviae,* and the labeled product of the Cy3-labeled genomic DNA derived from *Chlamydia psttaci* were obtained.

An ultrafiltration column, Microcon YM-30 (manufactured by Millipore Corp.) was set to the attached 1.5 mL tube, and then each of the above obtained labeled products of genomic DNA was placed on the column and centrifuged at 14,000 rpm for 4 minutes. The each concentrated solution was recovered in a microtube and dried thoroughly using a centrifugal vacuum drier (CentriVap concentrator; manufactured by Labconco Corp.).

(ii) Fragmentation process of the labeled products

To the labeled product of genomic DNA in dry state obtained in (i) of (4) above, a 40 μL of a solution having a composition of the final concentrations of 0.04 M Tris-acetate (pH 8.1), 0.1 M potassium acetate, and 0.03 M magnesium acetate tetrahydrate was added and mixed in suspension. After that, the suspensions were heat-treated at 94°C for 15 minutes, and the fragmentation products of each labeled genomic DNA with 100 to 300 bases were obtained.

The obtained solutions of Cy5-labeled product and Cy3-labeled product were each placed onto an ultrafiltration column of Microcon YM-10 (manufactured by Millipore Corp.), and centrifuged at 14,000 rpm for 4 minutes. After that, the concentrated solutions were recovered in the same microtube, and then dried thoroughly using a centrifugal vacuum drier (CentriVap concentrator; manufactured by Labconco Corp.). Subsequently, the following reagents were added to this microtube, and mixed in suspension, and the dry labeled products were dissolved.

ArrayHyb Hybridization buffer (manufactured by SIGMA-Aldrich); 40 μL;
Salmon sperm DNA (10 mg/mL); 0.5 μL;
Formamide; 5 μL
Total 40-50 μL

Through the above-described procedure, a mixed solution of Cy3Cy5-labeled products comprising the fragmentation product of the Cy5-labeled product out of the genomic DNA derived from *Chlamydophila caviae* and the fragmentation product of the Cy3-labeled product out of the control genomic DNA derived from *Chlamydia psttaci,* was obtained.

The obtained mixed solution of Cy3Cy5-labeled products was incubated at 95°C for 5 minutes, and kept at 70°C until use for hybridization.

(5) Microarray hybridization

On the microarray of the Whole Genome Shotgun clone Library of genomic DNA derived from *Chlamydophila caviae* obtained in the above step (3), the whole of the mixed solution of Cy3Cy5-labeled products obtained in the above-described (ii) of (4) was placed, and covered with a cover glass by keeping no air bubble remained inside. This microarray was set on a Hybri-cassette and placed on Kim Towel mat wetted with distilled water in a Tupperware and closed tightly, and was kept under light shielding at 65°C for not less than 8 hours to allow hybridization. After hybridization, the microarray was soaked in a solution of 2 x SSC containing 0.1% SDS together with cover glass at room temperature, and shook the microarray gently in the solution to remove the cover glass. Subsequently, after sequential washing with 1 x SSC solution containing 0.03% SDS (at 60°C) for 10 minutes, 0.2 x SSC solution (at 42°C) for 10 minutes and 0.05 x SSC solution (at room temperature) for 10 minutes, the microarray was transferred quickly to a new dry rack, and dried immediately by centrifugation at 800 rpm for 5 minutes.

(6) Measurement of fluorescence intensity: from signal detection to quantification

Using a fluorescence readout scanner GenePix 4000B (manufactured by Axon Instruments Inc.), the fluorescence intensity on the microarray obtained in the above (5) which received the microarray-hybridization treatment was measured. On this occasion, in order to analyze the results of competitive hybridization with Cy3-labeled product and Cy5-labeled product, detection of fluorescence was performed through 2 channels, namely 2ch (Cy3, Cy5) was detected.
The quantification of fluorescence signal (fluorescence detection data) was performed using DNASIS™-Array (DNA chip expression image analysis software; manufactured by Hitachi Software Engineering Co.), and according to the operational procedure of the software, automatic spot recognition, background calculation, and normalization of the fluorescence intensity ratio were carried out. In addition, by establishing a threshold limit line of reliability, and by avoiding the value lower than this line, a reliable normalized fluorescence intensity (ratio) was obtained.
Further, on the basis of the fluorescence intensity ratio of Cy3/Cy5 (Ratio) detected on the microarray, scatter chart (scatter plot) analysis was carried out according to the conventional procedure.
That is, when the fluorescence intensity ratio of Cy5 to Cy3 for a certain spot on the microarray is high, it indicates that the DNA fragment (PCR product) of the spot has been hybridized more strongly with the Cy5-labeled product, namely with the genomic DNA derived from *Chlamydophila caviae.* On the other hand, when the fluorescence intensity ratio of Cy5 to Cy3 for a certain spot on the microarray is low, it indicates that the DNA fragment of the spot has low specificity for the genomic DNA derived from *Chlamydophila caviae,* but cross reaction with the control genomic DNA derived from *Chlamydia psttaci* took place (hybridized with the control genomic DNA derived from *Chlamydia psttaci).*
By this method, the fluorescence intensity ratio of all the spot of the microarray was calculated, and the spots having high fluorescence intensity and having high fluorescence intensity ratio of Cy5 to Cy3 was selected.
Consequently, 6 clones which have hybridized more strongly with *Chlamydophila caviae* were selected as candidate clones.
(7) Determination of nucleotide sequence of the candidate clones Next, for the 6 candidate clones selected in the above (6), their nucleotide sequence were determined by the method described below.
Namely, using Big Dye Terminator kit (manufactured by Applied Biosystems Inc.), sequence analysis was carried out by the following procedure according to the protocol of the product.

Candidate DNA (candidate clone); 2 μL (100 ng)

M13 Primer M1; 1 μL (5 pmol)
Premix; 8 μL
To the above mixture, deionized and sterilized water was added to give a total volume of 20 μL, and then 30 cycles of the PCR under the following reaction conditions was carried out using the DNA Thermal Cycler (DNA Engine PTC200; manufactured by MJ Research Inc.).
96°C for 2 mien → (96°C for 10 sec → 50°C for 5 sec → 60°C for 4 min) x 25 → 4°C The obtained PCR products were purified using a gel filtration column (manufactured by QUIAGEN GmbH), and then, using a sequencer (BaseStation; manufactured by MJ Research Inc.), and according to the operation manual attached to the instrument, sequence (nucleotide sequence) mapping for all of nucleotide sequence of the candidate clones was completed.
The sequence information on the obtained candidate clone 01 to 06 was compared with the genome sequence of type strain of *Chlamydophila caviae* (type strain GenBank Acc No.AE015925) using the database (NCBI BLAST and CHEMICAL ABSTRACT). The results were shown in the following tables 4.
In Table 4, "position" shows the position of the nucleotide sequence of each candidate clone on the nucleotide sequence of the genomic gene of the type strain of *Chlamydophila caviae* (type strain GenBank Acc No.AE015925).
In addition, "ID" indicates clone ID No. which was named by the present inventor.

Table 4

| As is clear from Table 4, for the respective whole genome sequence of the selected candidate clone 01 to 06, there were areas of overlap in the reported nucleotide | | | |
|---|---|---|---|
| | **ID** | **positon (on GenBank Acc No=AE015925)** | **Size of oligonucleotide** |
| Candidate clone 01 | R08_4f | 1017881 ~ 1018913 | 1033 |
| Candidate clone 02 | R08_3d | 917102 ~ 918030 | 929 |
| Candidate clone 03 | R12_2a | 1151973 ~ 1153046 | 1074 |
| Candidate clone 04 | R12_4h | 631260 ~ 632334 | 1075 |
| Candidate clone 05 | R10_1g | 300776 ~ 305249 | 4474 |
| Candidate clone 06 | R10_12d | 922201 ~ 923189 | 989 |
| sequence of genomic gene of the type strain of *Chlamydophila caviae.* | | | |

On the other hand, for the respective whole genome sequence of the selected candidate clone 01 to 06, there was no overlapping area in the reported nucleotide sequence of closely related strain to *Chlamydophila caviae* (genus *Chlamydophila* other than *Chlamydophila caviae* or genus *Chlamydia).* From the facts described above, it was presumed that the nucleotide sequence of 6 clones of candidate were the areas of highly specific for *Chlamydophila caviae*

Example 2: Specificity evaluation of the candidate clone 02 for *Chlamydophila caviae*

(1) Synthesis of the primer of the present invention

Firstly, among 6 candidate clones determined in the above-described (7) of Example 1, based on the result of sequence (nucleotide sequence) analysis of the candidate clone 02 (clone ID=R08_3d), primer sequence for use in the PCR, namely, "5'-tcttcccgcctccttattct-3"' (SEQ ID NO:11; hereinafter referred to as R08_3d_Fw1), and "5'-gctgcttgtggggcaatc-3"' (SEQ ID NO:12; hereinafter referred to as R08_3d_Rv1) were designed using a primer design tool on the web, Primer 3 (Whitehead Institute for Biomedical Research).

In addition, nucleotide sequence of the candidate clone 02 obtained from the result of sequence analysis is the one shown in SEQ ID NO:2.

Next, the designed oligonucleotide was synthesized by the phosphoamidite method using ABI 392 DNA synthesizer. The synthetic procedure was carried out according to the operation manual provided by ABI, and the deprotection of various types of oligonucleotides was performed by heating aqueous ammonia solution of the oligonucleotide at 55°C for overnight.

Subsequently, the synthesized oligonucleotide was purified by anion-exchange column chromatography using Pharmacia FPLC. This synthetic oligonucleotide was used as a primer.

(2) Preparation of the probe of the present invention

The nucleotide sequence which is anticipated to be amplified by the PCR using R08_3d_Fw1 and R08_3d_Rv1 as primers is a nucleotide sequence shown in SEQ ID NO:31 (135 bases). And so, from the nucleotide sequence shown in SEQ ID NO:31, a sequence "5'-tcaacaagatattactgcggcaacacc-3"' to be used as a probe was designed, and an oligonucleotide having this sequence was synthesized (SEQ ID NO:40). An oligonucleotide probe having this sequence is hereinafter indicated as R08_3d_FwRv1-FAM. By binding a reporter dye FAM on the 5'-terminal and the reporter-quenching substance TAMRA on the 3'-terminal of this oligonucleotide, a labeled oligonucleotide probe of the present invention (TaqMan™ fluorescent probe; manufactured by Applied Bio-systems Japan) was obtained.

(3) Preparation of DNA sample for PCR

Each bacterium shown in the following Table 5 was cultured individually according to the conventional procedures, and then the purified genomic DNA was obtained using known nucleic acids purification method.

In addition, all of the bacteria listed in Table 5 were supplied by Professor Hiromi Kumon of School of Medicine, Okayama University.

Table 5

| species | strain |
|---|---|
| *Chlamydophila caviae* | GPIC<br>OK135 (Clinical isolate)<br>OKM112 (Clinical isolat<br>SC10 (Clinical isolate) |
| *Chlamydia trachomatis* | A (Serovar)<br>C (Serovar)<br>D (Serovar)<br>F (Serovar)<br>G (Serovar)<br>H (Serovar)<br>I (Serovar)<br>L1 (Serovar)<br>L2 (Serovar)<br>L3 (Serovar) |
| *Chlamydia pneumoniae* | TW183<br>YK41<br>KKpn15<br>KKpn1 |
| *Chlamydophila psittaci* | Cal10<br>Budgerigan-1<br>Izawa-1 |
| *Chlamydophila pecorum* | Maeda<br>E58 |

Each of the purified DNA obtained was adjusted to give final concentration of 1 ng/μL (in 10 mM Tris-HCl buffer, pH 8.9), and used as DNA sample for PCR.

(4) Real-time PCR

Using the R08_3d_Fw1 as a forward primer and the R08_3d_Rv1 as a reverse primer which were designed and synthesized in the above-described (1), the PCR was carried out as follows.

(i) Preparation of reaction solution for PCR

A 10 mM Tris-HCl buffer solution (pH 8.9) containing 1 μM each of forward primer R08_3d_Fw1 and reverse primer R08_3d_Rv1, 195 nM fluorescent labeled probe R08_3d_FwRv1_FAM prepared in the above (2), 1.5 mM $MgCl_2$, 80 mM KCl, 500 μg/mL BSA, 0.1% sodium cholate, 0.1% Triton X-100, 0.2 mM each of dATP, dCTP, dGTP and dTTP, and 40 unit/mL of Taq DNA polymerase (manufactured by Nippon Gene Co. Ltd.) was prepared and used as a reaction solution for PCR.

(ii) Real-time PCR

To 20 μL of the reaction solution for PCR prepared in the above-described (i) of (4), 1 μL of the DNA sample for PCR prepared in the above-described (3) was added and used as a sample for PCR.

This sample for PCR was placed in a glass capillary tube for quantitative PCR reaction (manufactured by Roche A.G.), and the real-time PCR was carried out using a specialized thermal cycler and detector for PCR (LightCycler2.0; manufactured by Roche A.G.).

That is, after keeping the temperature at 95°C for 10 minutes, a reaction cycle composed of heating at 95°C for 15 seconds and at 60°C for 1 minute was repeated for 50 cycles, and at every cycle, the fluorescence intensity derived from the reporter dye was measured. In addition, the fluorescence intensity was measured by using the use of a function of digitalizing relative fluorescent intensity ratio equipped to the thermal cycler used for the measurement, for each glass capillary tube used for measurement.

(5) Result

The obtained result of the real time PCR was summarized in Table 6.
Table 6

| species | strain | Determination |
|---|---|---|
| *Chlamydophila caviae* | GPIC | positive |
| | OK135 (Clinical isolate) | positive |
| | OKM112 (Clinical isolate) | positive |
| | SC10 (Clinical isolate) | positive |
| *Chlamydia trachomatis* | A (Serovar) | negative |
| | C (Serovar) | negative |
| | D (Serovar) | negative |
| | F (Serovar) | negative |
| | G (Serovar) | negative |
| | H (Serovar) | negative |
| | I (Serovar) | negative |
| | L1 (Serovar) | negative |
| | L2 (Serovar) | negative |
| | L3 (Serovar) | negative |
| *Chlamydia pneumoniae* | TW183 | negative |
| | YK41 | negative |
| | KKpn15 | negative |
| | KKpn1 | negative |
| *Chlamydophila psittaci* | Cal10 | negative |
| | Budgerigan-1 | negative |
| | Izawa-1 | negative |
| *Chlamydophila pecorum* | Maeda | negative |
| | E58 | negative |

In Table 6, the case where fluorescence signal was detected was indicated as "positive", and the case where fluorescence signal was not detected was indicated as "negative".

As is clear from Table 6, as a result of detection of the amplified primer extension product by the real-time PCR using the primer R08_3d_Fw1 and the primer R08_3d_Rv1 of the present invention, and using a labeled oligonucleotide probe (the probe of the present invention) having a sequence designed from the sequence of the region to be amplified, the fluorescence signal generated as the result of nucleic acid amplification was confirmed only when the PCR was carried out using genomic DNA sample derived from *Chlamydophila caviae* as a template, and determined as positive. On the other hand, when the real-time PCR was similarly performed using the combination of the same primer, and using genomic DNA derived from *Chlamydia* species other than *Chlamydophila caviae* as a template, the fluorescence signal could not be detected, and all were determined to be negative.

From the results obtained above, it turned out that, by the use of the oligonucleotide of the present invention as a primer for the PCR, *Chlamydophila caviae* could be detected specifically. In addition, since the detection by nucleic acid amplification such as PCR is expected to have high sensitivity, isolation of bacterium is not necessary, but the clinical specimen can be used directly for detection. Therefore, the detection of *Chlamydophila caviae* can be finished within a day at the longest, while it took several weeks for bacterial cultivation in the conventional detection method where detection is performed after bacterial cultivation.

Example 3: Test for minimum detection sensitivity

Using the real-time detection method, verification of detection sensitivity was carried out for a case where the candidate clone 02 sequence (clone ID = R08_3d) was targeted.

(1) Synthesis of the primer of the present invention

Using the same instrument and by the similar procedure as used in (1) of Example 1, oligonucleotides of R08_3d_Fw2 and R08_3d_Rv2 were synthesized. And these oligonucleotides were used as a primer.

(2) Preparation of DNA sample for PCR

By the same procedures as performed in (1) of Example 1, purified genomic DNA derived from *Chlamydophila caviae* was obtained from *Chlamydophila caviae* (SC-10 strain).
This DNA was dissolved in 10 mM Tris-HCl buffer, and quantity of the DNA in the sample was determined by measuring absorbance thereof The quantity of the DNA (copy number of the genome) in the sample was determined by comparing the obtained absorbance with the measurement value which was obtained by measuring absorbance in the same way using the known concentration of genomic DNA of a type strain of *Chlamydophila caviae* as a sample.
Subsequently, the DNA sample was diluted using 10 mM Tris-HCl buffer, pH 8.9, to a dilution series of $10^5$, $10^4$, $10^3$, $10^2$, 10, and 5 copies/μL, and used as a DNA sample for PCR.

(3) Real-time PCR

(i) Preparation of reaction solution for PCR

A 10 mM Tris-HCl buffer solution containing 300 nM each of the primer R08_3d_Fw2 and the primer R08_3d_Rv2 obtained in the above-described (1), 30 times dilution of the undiluted solution (final concentration was 30000 times dilution of the undiluted solution) of SYB™ Green I (product name of Molecular Probes Inc.), 1.5 mM $MgCl_2$, 80 mM KCl, 500 μg/mL BSA, 0.1% sodium cholate, 0.1% Triton X-100, 0.2 mM each of dATP, dCTP, dGTP and dTTP, and 40 unit/mL ofTaq DNA polymerase (manufactured by Nippon Gene Co. Ltd.) was prepared, and used as a reaction solution for PCR.

(ii) Real-time PCR

Using the DNA sample for PCR derived from *Chlamydophila caviae* prepared in the above-described (2) as a template DNA to be an amplification target in the PCR, the real-time PCR by the intercalation method was carried out as follows, and quantitative monitoring of fluorescence was carried out.
Firstly, to 20 μL of the reaction solution for PCR prepared in the above-described (i) of (3), 1 μL (1 ng) of the DNA sample for PCR derived from *Chlamydophila caviae* prepared in the above-described (2) was added, and used as a sample for PCR. This sample for PCR was placed in each well of a 96-well reaction plate (MicroAmp Optical 96-well Reaction Plate; manufactured by Applied Biosystems Japan Ltd.), and the real-time PCR was carried out using a specialized thermal cycler/detector for the TaqMan™ PCR (ABI 7500; manufactured by Applied Biosystems Japan Ltd.).
That is, after keeping the temperature at 95°C for 10 minutes, a reaction cycle composed of heating at 95°C for 15 seconds and 60°C for 1 minute was repeated for 40 cycles, and the fluorescence intensity derived from SYBR™ Green I which has intercalated in correlation with the quantity of the primer extension products was measured.
It should be noted that, fluorescence intensity was measured by using a function of digitalizing relative fluorescent intensity ratio equipped to the thermal cycler used for the measurement, for each of the 96 well reaction plates used for the measurement.

(4) Result

From experimental data obtained, a standard curve was made up according to conventional procedure commonly performed in the real-time PCR method.
That is, as to each concentration of the DNA samples for PCR, the fluorescence intensity derived from SYBR™ Green I (Rn, y-axis) was plotted for each cycle number of PCR (x-axis) to make up an amplification curve. After that, an Rn part where the fluorescence intensity amplified exponentially was selected, and a Threshold line (Th) was drawn. The crossing point of the Th with the fluorescence intensity from each DNA sample for PCR was defined as Threshold cycle (Ct). After that, the Ct value (y-axis) was plotted for the copy number of the genome of each used DNA sample for PCR (x-axis), and the approximated curve obtained for each Ct was used as a standard curve. The standard curve obtained is shown in Fig. 1.

In Fig. 1, the approximation formula of the obtained approximated curve is as follows:

$$y = -3.720x + 38.26,$$

$$R^2 = 0.998$$

In consequence, from the fact that the fluorescence was detected by the real-time PCR, it turned out that *Chlamydophila caviae* can be detected by performing the real-time PCR using the oligonucleotide involved in the present invention as a primer.

In addition, it also turns out that, as the standard curve has become available, quantitative determination of *Chlamydophila caviae* is possible by the real-time PCR using the primer and the probe of the present invention.

Further, as is clear from Fig. 2, it turns out that the real-time PCR method using the primer and the probe of the present invention can detect *Chlamydophila caviae* even under the condition where only 5 copies of the genomic DNA of *Chlamydophila caviae* are present as initial quantity.

Furthermore, in the case where the real-time PCR method is applied, quantitative determination of the initial quantity of template DNA can be performed accurately, because the fluorescence intensity is monitored in real time, and therefore, the method is effective for the determination of *Chlamydophila caviae.*

Example 4: Evaluation of *Chlamydophila caviae* specificity of the other candidate clone

(1) Synthesis of the primer of the present invention

Based on the result of sequence (nucleotide sequence) analysis of the 6 candidate clones determined in (7) of Example 1, the primer sequence for the PCR amplification detection was designed from the nucleotide sequence of each candidate clone, using a primer design tool on the web, Primer 3 (Whitehead Institute for Biomedical Research).

Next, the designed oligonucleotide was synthesized and purified by the same method as used in (1) of Example 2. This synthesized oligonucleotide was used as a primer of the present invention.

Name of each candidate sequence, SEQ ID NO of nucleotide sequence of the candidate clone, name of the primer designed based on the nucleotide sequence of the candidate clone (named by the present inventor) and SEQ ID NO of the nucleotide sequence thereof, combination of forward primer and reverse primer used in the subsequent PCR were shown collectively in Table 7. In addition, the clone ID number of each candidate clone was shown in parentheses under the name of the candidate sequence.

Table 7

| Candidate clone | | Designed primer | | | | |
|---|---|---|---|---|---|---|
| Name | SEQ ID NO | Combination number | Forward primer | | Reverse primer | |
| | | | name | SEQ ID NO | Name | SEQ ID NO |
| Candidate clone 01 (R08_4f) | 1 | 1 | R08_4f_Fw1 | 7 | R08_4f_Rv1 | 8 |
| | | 2 | R08_4f_Fw2 | 9 | R08_4f_Rv2 | 10 |
| Candidate clone 02 (R08_3d) | 2 | 3 | R08_3d_Fw1 | 11 | R08_3d_Rv1 | 12 |
| | | 4 | R08_3d_Fw2 | 13 | R08_3d_Rv2 | 14 |
| Candidate clone 03 (R12_2a) | 3 | 5 | R12_2a_Fw1 | 15 | R12_2a_Rv1 | 16 |
| | | 6 | R12_2a_Fw2 | 17 | R12_2a_Rv2 | 18 |
| Candidate clone 04 (R12_4h) | 4 | 7 | R12_4h_Fw1 | 19 | R12_4h_Rv1 | 20 |
| | | 8 | R12_4h_Fw2 | 21 | R12_4h_Rv2 | 22 |

(continued)

| Candidate clone | | Designed primer | | | | | |
|---|---|---|---|---|---|---|---|
| Name | SEQ ID NO | Combination number | Forward primer | | Reverse primer | |
| | | | name | SEQ ID NO | Name | SEQ ID NO |
| Candidate clone 05 (R10_1g) | 5 | 9 | R10_1g_Fw1 | 23 | R10_1g_Rv1 | 24 |
| | | 10 | R10_1g_Fw2 | 25 | R10_1g_Rv2 | 26 |
| Candidate clone 06 (R10_12d) | 6 | 11 | R10_12d_Fw1 | 27 | R10_12d_Rv1 | 28 |

(2) Preparation of the DNA sample for PCR

Among the bacteria listed in Table 5 which were used in Example 2, *Chlamydophila caviae* (GPIC strain), *Chlamydophila psttaci* (Ca110), *Chlamydophila pneumoniae* (TW183 strain), *Chlamydia trachomatis* (D strain (Serovar)) were used, and the purified genomic DNA samples were prepared by the same method as described in (1) of Example 1.

In addition, the concentration of each DNA sample was adjusted using 10 mM Tris-HCl buffer solution (pH 8.9) so that the final concentration might be set to $10^4$, $10^3$ copies /$\mu$L (sufficient quantities to be detected each chlamydia), and used as a DNA sample for PCR.

(3) Real-time PCR

The real-time PCR was carried out by the same method as performed in (3) (ii) of Example 2 except for using the primer prepared in the above-described (1) in combination as described in the above Table 7 and using the DNA sample derived from each bacterium as a template.

(4) Melting curve analysis

As to the product respectively amplified for each DNA sample, melting curve was depicted by plotting the melting temperature of the primer extension product (double-stranded DNA) as horizontal axis and the first derivation (variation) of fluorescence intensity as vertical axis, and then detection of peak was examined.

(5) Result

The results were shown in Table 8.

Table 8

| clone ID | primer ID | croos-reation with Chlamydia species (copy/react) | | | | | | | |
|---|---|---|---|---|---|---|---|---|---|
| | | C caviae | | C psittaci | | C pneumoniae | | C trachomatis | |
| | | 1 00E+04 | 1 00E+03 | 1 00E+04 | 1 00E+03 | 1 00E+04 | 1 00E+03 | 1 00E+04 | 1 00E+03 |
| Candidate clone 01 R08_4f | R08_4f_ Fw1 & R08_4f_ Rv1 | + | + | - | - | - | - | - | - |
| | R08_4f_ Fw2 & R08_4f_ Rv2 | + | + | - | - | - | - | - | - |
| Candidate clone 02 R08_3d | R08_3d_ Fw1 & R08_3d_ Rv1 | + | + | - | - | - | - | - | - |

(continued)

| clone ID | primer ID | 1 00E+04 | 1 00E+03 | 1 00E+04 | 1 00E+03 | 1 00E+04 | 1 00E+03 | 1 00E+04 | 1 00E+03 |
|---|---|---|---|---|---|---|---|---|---|
| | R08_3d_ Fw2 & R08_3d_ Rv2 | + | + | - | - | - | - | - | - |
| Candidate clone 03 R12_2a | R12_2a_ Fw1 & R12_2a_ Rv1 | + | + | - | - | - | - | - | - |
| | R12_2a_ Fw2 & R12_2a_ Rv2 | + | + | - | - | - | - | - | - |
| Candidate clone 04 R12_4h | R12_4h_ Fw1 & R12- 4h_ Rv1 | + | + | - | - | - | - | - | - |
| | R12_4h_ Fw2 & R12_4h_ Rv2 | + | + | - | - | - | - | - | - |
| Candidate clone 05 R10_1g | R10_1g_ Fw1 & R10_1g_ Rv1 | + | + | - | - | - | - | - | - |
| | R10_1g_ Fw2 & R10_1g_ Rv2 | + | + | - | - | - | - | - | - |
| Candidate clone 06 R10_12d | R10_12d_ Fw1 & R10_12d_ Fw1 | + | + | - | - | - | - | - | - |

On the list, for example, 1.00E+04 shows the case where the concentration of DNA sample is $10^4$ copies/μL.

In Table 8, (+) shows the case where a peak is detected in melting curve analysis, and (-) shows the case where a peak was not detected in melting curve analysis, respectively.

As is clear from Table 8, as the result of the real-time PCR carried out using the combination of the primer of the present invention listed in Table 8, in any combination of the primers is used, only the case when the real-time PCR was carried out using DNA sample derived from *Chlamydophila caviae* as a template, the fluorescence generated as the result of nucleic acid amplification was confirmed, and thus it could be determined as positive.

On the other hand, when the real-time PCR was carried out in the same way using the DNA derived from closely related bacterial strain other than *Chlamydophila caviae* as a template, and using any combination of the same primers listed above, relevant fluorescence was not confirmed, and all cases were determined as negative. From the results described above, it is understood that out that according to the detection method of the present invention, occurrence of false positive results can be avoided, and *Chlamydophila caviae* can be detected specifically.

INDUSTRIAL APPLICABILITY

According to the method for detection of *Chlamydophila caviae* using the primer and/or the probe of the present invention, the detection of *Chlamydophila caviae* can be performed more rapidly and with high precision compared with a conventional bacterial species identification method performed by culture examination on a specific medium. In addition, the method for detection of *Chlamydophila caviae* of the present invention can exclude any false-positive result for the diagnosis and can also detect and diagnose *Chlamydophila caviae* with high precision. Further, the method for detection of *Chlamydophila caviae* of the present invention can quantify the *Chlamydophila caviae* cell.
An oligonucleotide which comprises a part or the entire of a nucleotide sequence selected from SEQ ID NO:1, SEQ ID NO:2, SEQ ID NO:3, SEQ ID NO:4, SEQ ID NO:5 and SEQ ID NO:6 (wherein, the characters of A, C, G and T represent adenine, cytosine, guanine and thymine, respectively; and T at an arbitrary position may be replaced by uracil (U); and hereinafter, same as above), or a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6, and which is capable of hybridizing with the nucleotide sequence of a *Chlamydophila caviae* gene.
The oligonucleotide according as defined above, wherein:

the oligonucleotide which comprises a part of the nucleotide sequence selected from SEQ ID NO:1, SEQ ID NO:2, SEQ ID NO:3, SEQ ID NO:4, SEQ ID NO:5 and SEQ ID NO:6 is the one which comprises a part or the entire of a nucleotide sequence selected from SEQ ID NO:7 to 39; and
the oligonucleotide which comprises a part of the nucleotide sequence complementary to the nucleotide sequence selected from SEQ ID NO:1, SEQ ID NO:2, SEQ ID NO:3, SEQ ID NO:4, SEQ ID NO:5 and SEQ ID NO:6 is the one which comprises a part or the entire of a nucleotide sequence complementary to the oligonucleotide comprising the nucleotide sequence selected from SEQ ID NO:7 to 39.

The oligonucleotide as defined above, wherein:

the oligonucleotide which comprises a part of the nucleotide sequence shown in SEQ ID NO:1 is the one which comprises a part or the entire of a nucleotide sequence selected from SEQ ID NO:7 to 10 and SEQ ID NO:29 to 30;
the oligonucleotide which comprises a part of the nucleotide sequence shown in SEQ ID NO:2 is the one which comprises a part or the entire of a nucleotide sequence selected from SEQ ID NO:11 to 14 and SEQ ID NO:31 to 32;
the oligonucleotide which comprises a part of the nucleotide sequence shown in SEQ ID NO:3 is the one which comprises a part or the entire of a nucleotide sequence selected from SEQ ID NO:15 to 18 and SEQ ID NO:33 to 34;
the oligonucleotide which comprises a part of the nucleotide sequence shown in SEQ ID NO:4 is the one which comprises a part or the entire of a nucleotide sequence selected from SEQ ID NO:19 to 22 and SEQ ID NO:35 to 36;
the oligonucleotide which comprises a part of the nucleotide sequence shown in SEQ ID NO:5 is the one which comprises a part or the entire of a nucleotide sequence selected from SEQ ID NO:23 to 26 and SEQ ID NO:37 to 38;
the oligonucleotide which comprises a part of the nucleotide sequence shown in SEQ ID NO:6 is the one which comprises a part or the entire of a nucleotide sequence selected from SEQ ID NO:27 to 28 and SEQ ID NO:39; and
the oligonucleotide which comprises a part of a sequence complimentary to the nucleotide sequence selected from SEQ ID NO:1, SEQ ID NO:2, SEQ ID NO:3, SEQ ID NO:4, SEQ ID NO:5 and SEQ ID NO:6 is the one which comprises a part or the entire of a sequence complementary to the nucleotide sequence selected from SEQ ID NO: 7 to 39.
A primer for detection of *Chlamydophila caviae* comprising an oligonucleotide which comprises a part or the entire of a nucleotide sequence selected from SEQ ID NO:1, SEQ ID NO:2, SEQ ID NO:3, SEQ ID NO:4, SEQ ID NO:5 and SEQ ID NO:6, or a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6, and which is capable of hybridizing with the nucleotide sequence of *a Chlamydophila caviae* gene.

The primer as defined above, wherein:

the oligonucleotide which comprises a part of a nucleotide sequence shown in SEQ ID NO:1 is the one which comprises a part or the entire of a nucleotide sequence selected from SEQ ID NO:7 to 10 and SEQ ID NO:29 to 30;
the oligonucleotide which comprises a part of a nucleotide sequence shown in SEQ ID NO:2 is the one which comprises a part or the entire of a nucleotide sequence selected from SEQ ID NO:11 to 14 and SEQ ID NO:31 to 32;
the oligonucleotide which comprises a part of a nucleotide sequence shown in SEQ ID NO:3 is the one which comprises a part or the entire of a nucleotide sequence selected from SEQ ID NO:15 to 18 and SEQ ID NO:33 to 34;
the oligonucleotide which comprises a part of a nucleotide sequence shown in SEQ ID NO:4 is the one which comprises a part or the entire of a nucleotide sequence selected from SEQ ID NO:19 to 22 and SEQ ID NO:35 to 36;
the oligonucleotide which comprises a part of a nucleotide sequence shown in SEQ ID NO:5 is the one which comprises a part or the entire of a nucleotide sequence selected from SEQ ID NO:23 to 26 and SEQ ID NO:37 to 38; and
the oligonucleotide which comprises a part of a nucleotide sequence shown in SEQ ID NO:6 is the one which comprises a part or an entire of a nucleotide sequence selected from SEQ ID NO:27 to 28 and SEQ ID NO:39; and
the oligonucleotide which comprises a part of a sequence complimentary to the nucleotide sequence selected from SEQ ID NO:1, SEQ ID NO:2, SEQ ID NO:3, SEQ ID NO:4, SEQ ID NO:5 and SEQ ID NO:6 is the one which comprises a part or the entire of a sequence complementary to the nucleotide sequence selected from SEQ ID NO: 7 to 39.

The primer as defined above, wherein the number of nucleotide constituting the primer is 10 to 50.
The primer as defined above, wherein the primer is labeled with a labeling substance. The primer as defined above, wherein the labeling substance is the one selected from a radioisotope, an enzyme, a fluorescent substance, a luminescent substance or biotin.
A probe for detection of *Chlamydophila caviae* comprising an oligonucleotide which comprises a part or the entire of a nucleotide sequence selected from SEQ ID NO:1, SEQ ID NO:2, SEQ ID NO:3, SEQ ID NO:4, SEQ ID NO:5 and SEQ ID NO:6, or a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6, and which is capable of hybridizing with the nucleotide sequence of *a Chlamydophila caviae* gene.
The probe as defined above, wherein the probe is labeled with a labeling substance.
The probe as defined above, wherein the labeling substance is the one selected from a radioisotope, an enzyme, a fluorescent substance, a luminescent substance or biotin.
The probe as defined above, wherein the 5'-terminal of the probe is labeled with a reporter fluorescent dye and the 3'-terminal of the probe is labeled with a quencher dye.
A method for detection of *Chlamydophila caviae,* characterized in that an oligonucleotide which comprises a part or the entire of a nucleotide sequence selected from SEQ ID NO:1, SEQ ID NO:2, SEQ ID NO:3, SEQ ID NO:4, SEQ ID NO: 5 and SEQ ID NO:6, or a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6, and which is capable of hybridizing with the nucleotide sequence of *a Chlamydophila caviae* gene is used as a primer and/or a probe.
The detection method as defined above, characterized in that the nucleic acid amplification reaction is carried out using as a primer an oligonucleotide which comprises a part or the entire of a nucleotide sequence selected from SEQ ID NO: 1, SEQ ID NO:2, SEQ ID NO:3, SEQ ID NO:4, SEQ ID NO:5 and SEQ ID NO:6, or a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6, and which is capable of hybridizing with the nucleotide sequence of *a Chlamydophila caviae* gene, and using nucleic acid in a sample as a template, and the obtained primer extension product is detected.
The detection method as defined above, wherein the method further comprising using a labeled probe prepared by labeling an oligonucleotide which comprises a part or the entire of a nucleotide sequence selected from SEQ ID NO:1, SEQ ID NO:2, SEQ ID NO:3, SEQ ID NO:4, SEQ ID NO:5 and SEQ ID NO:6, or a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6, and which is capable of hybridizing with the nucleotide sequence of *a Chlamydophila caviae* gene with a labeling substance.
The detection method as defined above, comprising
carrying out the nucleic acid amplification reaction using as a primer an oligonucleotide which comprises a part or the entire of a nucleotide sequence selected from SEQ ID NO:1, SEQ ID NO:2, SEQ ID NO:3, SEQ ID NO:4, SEQ ID NO: 5 and SEQ ID NO:6, or a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6, and which is capable of hybridizing with the nucleotide sequence of *a Chlamydophila caviae* gene, and using a labeled probe prepared by labeling an oligonucleotide which comprises a part or the entire of a nucleotide sequence selected from SEQ ID NO:1, SEQ ID NO:2, SEQ ID NO:3, SEQ ID NO:4, SEQ ID NO:5 and SEQ ID NO:6, or a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6, and which is capable of hybridizing with the nucleotide sequence of *a Chlamydophila caviae* gene with a labeling substance, and further using the nucleic acid in a sample as a template, and
detecting the signal derived from the labeling substance released from the labeled probe.
The detection method as defined above, characterized by comprising the following steps:

(1) carrying out a nucleic acid amplification reaction using as a primer an oligonucleotide which comprises a part or the entire of a nucleotide sequence selected from SEQ ID NO:1, SEQ ID NO:2, SEQ ID NO:3, SEQ ID NO:4, SEQ ID NO:5 and SEQ ID NO:6, or a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6, and which is capable of hybridizing with the nucleotide sequence of *a Chlamydophila caviae* gene, and using the nucleic acid in a sample as a template; and
(2) carrying out an electrophoresis of the primer extension product obtained in (1), and determining the presence of *Chlamydophila caviae* based on the result of the electrophoresis.

The detection method as defined above, wherein the sample is determined as positive for *Chlamydophila caviae* in either one of the following cases:

(1) a case when, after electrophoresis, the electrophoretic fraction obtained is examined for the presence of a primer extension product having objective base pair size, and the primer extension product having the objective base pair size is confirmed;
(2) a case when, after electrophoresis, a hybridization of the electrophoretic fraction obtained is carried out with a labeled probe prepared by labeling an oligonucleotide which comprises a part or the entire of a nucleotide sequence selected from SEQ ID NO:1, SEQ ID NO:2, SEQ ID NO:3, SEQ ID NO:4, SEQ ID NO:5 and SEQ ID NO:6, or a part or the entire of a sequence complementary to the nucleotide sequence selected from SEQ ID NO:1, SEQ ID NO: 2, SEQ ID NO:3, SEQ ID NO:4, SEQ ID NO:5 and SEQ ID NO:6, and which is capable of hybridizing with the nucleotide sequence of a *Chlamydophila caviae* gene with a labeling substance, and a fraction hybridized with the labeled probe is confirmed by detecting a signal derived from the labeled probe. The detection method for *Chlamydophila caviae* as defined above, wherein the primer has been labeled with a labeling substance; and wherein the polymerase chain reaction is carried out using the primer and the nucleic acid in a sample as a template, thereafter a signal derived from the obtained primer extension product is measured.

The detection method as defined above, wherein, after the nucleic acid amplification chain reaction is carried out, and free labeled primer is removed, then a signal derived from the primer extension product is measured.
The detection method for *Chlamydophila caviae* as defined above, characterized in that, an oligonucleotide which comprises a part or an entire of a nucleotide sequence selected from SEQ ID NO:1, SEQ ID NO:2, SEQ ID NO:3, SEQ ID NO:4, SEQ ID NO:5 and SEQ ID NO:6, or a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6, and which is capable of hybridizing with the nucleotide sequence of *a Chlamydophila caviae* gene, is labeled with a labeling substance and used as a labeled probe;
the labeled probe is hybridized with the nucleic acid in a sample; free labeled probe is removed;
and then a signal derived from the hybridized complex is detected.
A reagent kit for detection of *Chlamydophila caviae* comprising an oligonucleotide which comprises a part or the entire of a nucleotide sequence selected from SEQ ID NO:1, SEQ ID NO:2, SEQ ID NO:3, SEQ ID NO:4, SEQ ID NO:5 and SEQ ID NO:6, or a part or the entire of a sequence complementary to the nucleotide sequence selected from SEQ ID NO:1, SEQ ID NO:2, SEQ ID NO:3, SEQ ID NO:4, SEQ ID NO:5 and SEQ ID NO:6, and which is capable of hybridizing with the nucleotide sequence of *a Chlamydophila caviae* gene, as a primer and/or a probe.

## Claims

1. A primer for detection of *Chlamydophila caviae,* comprising:

    an oligonucleotide designed from a nucleotide sequence consisting of SEQ ID NO:5, SEQ ID NO:2, SEQ ID NO:3, SEQ ID NO:4 or SEQ ID NO:6, or from the sequence complementary to the nucleotide sequence consisting of SEQ ID NO:5, SEQ ID NO:2, SEQ ID NO:3, SEQ ID NO:4 or SEQ ID NO:6,
    wherein the oligonucleotide consists of 10 to 35 bases,
    wherein the oligonucleotide is capable of hybridizing with the nucleotide sequence of a *Chlamydophila caviae* gene.

2. A primer according to claim 1, wherein

    the oligonucleotide designed from the nucleotide sequence consisting of SEQ ID NO:5, SEQ ID NO:2, SEQ ID NO:3, SEQ ID NO:4 or SEQ ID NO:6: comprises an oligonucleotide which comprises a nucleotide sequence selected from SEQ ID NO:23 to 26, wherein the oligonucleotide is a part of a nucleotide sequence shown SEQ ID NO:5;
    an oligonucleotide which comprises a nucleotide sequence selected from SEQ ID NO:11 to 14, wherein the oligonucleotide is a part of a nucleotide sequence shown SEQ ID NO:2;
    an oligonucleotide which comprises a nucleotide sequence selected from SEQ ID NO:15 to 18, wherein the oligonucleotide is a part of a nucleotide sequence shown SEQ ID NO:3;
    an oligonucleotide which comprises a nucleotide sequence selected from SEQ ID NO:19 to 22, wherein the oligonucleotide is a part of a nucleotide sequence shown SEQ ID NO:4; or
    an oligonucleotide which comprises a nucleotide sequence selected from SEQ ID NO:27 to 28, wherein the oligonucleotide is a part of a nucleotide sequence shown SEQ ID NO:6; and
    the oligonucleotide designed from the sequence complementary to the nucleotide sequence consisting of SEQ ID NO:5, SEQ ID NO:2, SEQ ID NO:3, SEQ ID NO:4 or SEQ ID NO:6 comprises the oligonucleotide which comprises a sequence complimentary to the nucleotide sequence selected from SEQ ID NO:23 to 26, 11 to 14, 15 to 18, 19 to 22, 27 to 28.

3. The primer according to claim 2, wherein the primer is labeled with a labeling substance, preferably the labeling substance is selected from a radioisotope, an enzyme, a fluorescent substance, a luminescent substance or biotin.

4. A probe for detection of *Chlamydophila caviae,* comprising:

    an oligonucleotide designed from a nucleotide sequence consisting of SEQ ID NO:5, SEQ ID NO:2, SEQ ID NO:3, SEQ ID NO:4 or SEQ ID NO:6 or from the sequence complementary to the nucleotide sequence shown in SEQ ID NO:5, SEQ ID NO:2, SEQ ID NO:3, SEQ ID NO:4 or SEQ ID NO:6; wherein the oligonucleotide consisting of the nucleotide sequence shown in SEQ ID NO:5, SEQ ID NO:2, SEQ ID NO:3, SEQ ID NO:4 or SEQ ID NO:6, or the sequence complementary to the nucleotide sequence shown in SEQ ID NO:5, SEQ ID NO:2, SEQ ID NO:3, SEQ ID NO:4 or SEQ ID NO:6; or
    15 to 40 or 100 to 600 bases of the nucleotide sequence shown in SEQ ID NO:5, SEQ ID NO:2, SEQ ID NO: 3, SEQ ID NO:4 or SEQ ID NO:6, or 15 to 40 or 100 to 600 bases of the sequence complementary to the nucleotide sequence shown in SEQ ID NO:5, SEQ ID NO:2, SEQ ID NO:3, SEQ ID NO:4 or SEQ ID NO:6;

    wherein the oligonucleotide is capable of hybridizing with the nucleotide sequence of a *Chlamydophila caviae* gene.

5. The probe according to claim 4, wherein

    the oligonucleotide designed from the nucleotide sequence consisting of SEQ ID NO:5, SEQ ID NO:2, SEQ ID NO:3, SEQ ID NO:4 or SEQ ID NO:6 comprises an oligonucleotide which comprises a nucleotide sequence selected from SEQ ID NO:5, NO:23 to 26 and SEQ ID NO:37 to 38 wherein the oligonucleotide is a part of a nucleotide sequence shown SEQ ID NO:5;
    an oligonucleotide which comprises a nucleotide sequence selected from SEQ ID NO:2, SEQ ID NO:11 to 14, SEQ ID NO:31 to 32, and SEQ ID NO:40 wherein the oligonucleotide is a part of a nucleotide sequence shown SEQ ID NO:2;
    an oligonucleotide which comprises a nucleotide sequence selected from SEQ ID NO:3, SEQ ID NO:15 to 18 and SEQ ID NO:33 to 34 wherein the oligonucleotide is a part of a nucleotide sequence shown SEQ ID NO:3;

an oligonucleotide which comprises a nucleotide sequence selected from SEQ ID NO:4, SEQ ID NO:19 to 22 and SEQ ID NO:35 to 36 wherein the oligonucleotide is a part of a nucleotide sequence shown SEQ ID NO:4; or an oligonucleotide which comprises a nucleotide sequence selected from SEQ ID NO:6, SEQ ID NO:27 to 28 and SEQ ID NO:39 wherein the oligonucleotide is a part of a nucleotide sequence shown SEQ ID NO:6; and the oligonucleotide designed from the sequence complementary to the nucleotide sequence shown in SEQ ID NO:5, SEQ ID NO:2, SEQ ID NO:3, SEQ ID NO:4 or SEQ ID NO:6 comprises an oligonucleotide which comprises a sequence complementary to the nucleotide sequence selected from SEQ ID NO:5, 23 to 26, 37 to 38, 2, 11 to 14, 31 to 32, 3, 15 to 18, 33 to 34, 4, 19 to 22, 35 to 36, 6, 27 to 28, 39.

**6.** The probe according to claim 4, wherein

(i) the probe is labeled with a labeling substance, preferably the labeling substance is the one selected from a radioisotope, an enzyme, a fluorescent substance, a luminescent substance or biotin, or
(ii) the 5'-terminal of the probe is labeled with a reporter fluorescent dye and the 3'-terminal of the probe is labeled with a quencher dye.

**7.** A method for detection of *Chlamydophila caviae,* comprising using the primer comprising an oligonucleotide designed from a nucleotide sequence consisting of SEQ ID NO:5, SEQ ID NO:2, SEQ ID NO:3, SEQ ID NO:4 or SEQ ID NO:6, or from the sequence complementary to the nucleotide sequence consisting of SEQ ID NO:5, SEQ ID NO:2, SEQ ID NO:3, SEQ ID NO:4 or SEQ ID NO:6,
wherein the oligonucleotide consists of 10 to 35 bases,
wherein the oligonucleotide is capable of hybridizing with the nucleotide sequence of a *Chlamydophila caviae* gene, and/or
the probe comprising an oligonucleotide designed from a nucleotide sequence consisting of SEQ ID NO:5, SEQ ID NO:2, SEQ ID NO:3, SEQ ID NO:4 or SEQ ID NO:6 or from the sequence complementary to the nucleotide sequence shown in SEQ ID NO:5, SEQ ID NO:2, SEQ ID NO:3, SEQ ID NO:4 or SEQ ID NO:6:
wherein the oligonucleotide consisting of
the nucleotide sequence shown in SEQ ID NO:5, SEQ ID NO:2, SEQ ID NO:3, SEQ ID NO:4 or SEQ ID NO:6, or the sequence complementary to the nucleotide sequence shown in SEQ ID NO:5, SEQ ID NO:2, SEQ ID NO:3, SEQ ID NO:4 or SEQ ID NO:6; or 15 to 40 or 100 to 600 bases of the nucleotide sequence shown in SEQ ID NO:5, SEQ ID NO:2, SEQ ID NO:3, SEQ ID NO:4 or SEQ ID NO:6, or 15 to 40 or 100 to 600 bases of the sequence complementary to the nucleotide sequence shown in SEQ ID NO:5, SEQ ID NO:2, SEQ ID NO:3, SEQ ID NO:4 or SEQ ID NO:6;
wherein the oligonucleotide is capable of hybridizing with the nucleotide sequence of *a Chlamydophila caviae* gene.

**8.** The method according to claim 7, **characterized in that** the nucleic acid amplification reaction is carried out using the primer, and using nucleic acid in a sample as a template, and the obtained primer extension product is detected.

**9.** The method according to claim 8, wherein the method further comprising using a labeled probe prepared by labeling the probe with a labeling substance.

**10.** The method according to claim 8, comprising
carrying out the nucleic acid amplification reaction using the primer, and using a labeled probe prepared by labeling the probe with a labeling substance, and further using the nucleic acid in a sample as a template, and
detecting the signal derived from the labeling substance released from the labeled probe.

**11.** The method according to claim 7, **characterized by** comprising the following steps:

(1) carrying out a nucleic acid amplification reaction using the primer, and using the nucleic acid in a sample as a template; and
(2) carrying out an electrophoresis of the primer extension product obtained in (1), and determining the presence of *Chlamydophila caviae* based on the result of the electrophoresis.

**12.** The method according to claim 11, wherein the sample is determined as positive for *Chlamydophila caviae* in either one of the following cases:

(1) a case when, after electrophoresis, the electrophoretic fraction obtained is examined for the presence of a primer extension product having objective base pair size, and the primer extension product having the objective base pair size is confirmed;
(2) a case when, after electrophoresis, a hybridization of the electrophoretic fraction obtained is carried out with a labeled probe prepared by labeling the probe with a labeling substance, and a fraction hybridized with the labeled probe is confirmed by detecting a signal derived from the labeled probe.

**13.** The method according to claim 7, wherein the primer is labeled with a labeling substance; and wherein the nucleic acid amplification reaction is carried out using the primer and the nucleic acid in a sample as a template, thereafter a signal derived from the obtained primer extension product is measured, preferably, after the nucleic acid amplification reaction is carried out, and free labeled primer is removed, then a signal derived from the primer extension product is measured.

**14.** The method according to claim 7, wherein the probe is labeled with a labeling substance; the labeled probe is hybridized with the nucleic acid in a sample; free labeled probe is removed; and then a signal derived from the hybridized complex is detected.

**15.** A reagent kit for detection of *Chlamydophila caviae* comprising the primer comprising an oligonucleotide designed from a nucleotide sequence consisting of SEQ ID NO:5, SEQ ID NO:2, SEQ ID NO:3, SEQ ID NO:4 or SEQ ID NO:6, or from the sequence complementary to the nucleotide sequence consisting of SEQ ID NO:5, SEQ ID NO:2, SEQ ID NO:3, SEQ ID NO:4 or SEQ ID NO:6, wherein the oligonucleotide consists of 10 to 35 bases,
wherein the oligonucleotide is capable of hybridizing with the nucleotide sequence of a *Chlamydophila caviae* gene, and/or
the probe comprising an oligonucleotide designed from a nucleotide sequence consisting of SEQ ID NO:5, SEQ ID NO:2, SEQ ID NO:3, SEQ ID NO:4 or SEQ ID NO:6 or from the sequence complementary to the nucleotide sequence shown in SEQ ID NO:5, SEQ ID NO:2, SEQ ID NO:3, SEQ ID NO:4 or SEQ ID NO:6:
wherein the oligonucleotide consisting of
the nucleotide sequence shown in SEQ ID NO:5, SEQ ID NO:2, SEQ ID NO:3, SEQ ID NO:4 or SEQ ID NO:6, or the sequence complementary to the nucleotide sequence shown in SEQ ID NO:5, SEQ ID NO:2, SEQ ID NO:3, SEQ ID NO:4 or SEQ ID NO:6; or 15 to 40 or 100 to 600 bases of the nucleotide sequence shown in SEQ ID NO:5, SEQ ID NO:2, SEQ ID NO:3, SEQ ID NO:4 or SEQ ID NO:6, or 15 to 40 or 100 to 600 bases of the sequence complementary to the nucleotide sequence shown in SEQ ID NO:5, SEQ ID NO:2, SEQ ID NO:3, SEQ ID NO:4 or SEQ ID NO:6;
wherein the oligonucleotide is capable of hybridizing with the nucleotide sequence of *a Chlamydophila caviae* gene.

[Fig.1]

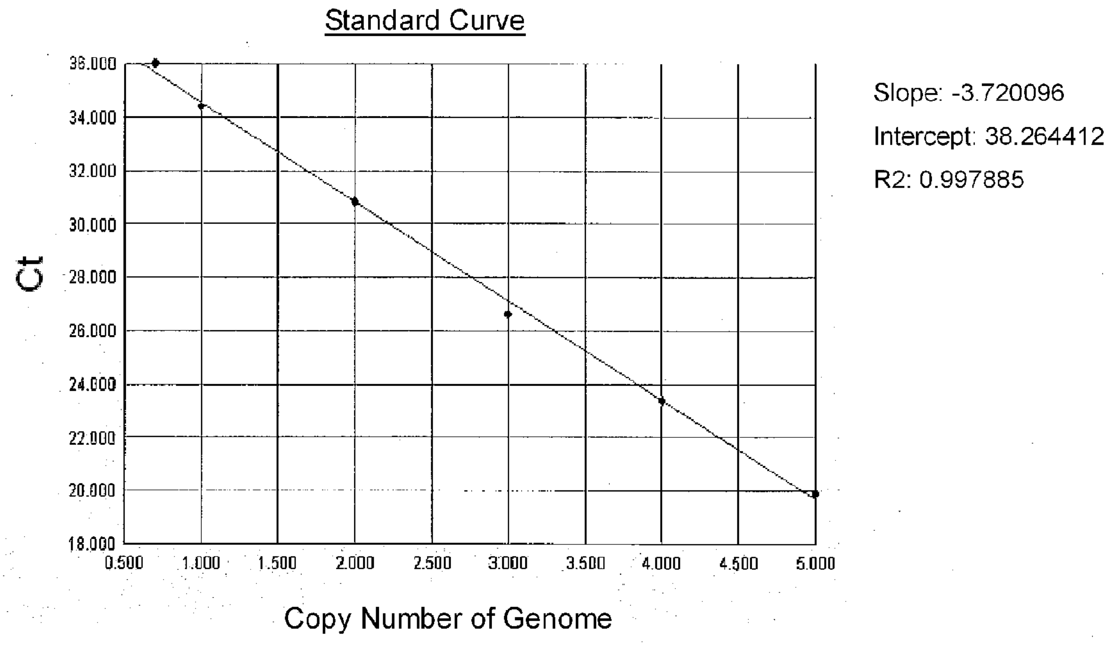
Standard Curve
Ct
36.000
34.000
32.000
30.000
28.000
26.000
24.000
22.000
20.000
18.000
0.500
1.000
1.500
2.000
2.500
3.000
3.500
4.000
4.500
5.000
Copy Number of Genome
Slope: -3.720096
Intercept: 38.264412
R2: 0.997885

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5232829 A
- JP 11155589 A
- JP 60500717 A
- JP 60050717 A
- JP 4211399 A
- JP 8019394 A
- US 5210015 A
- US P5538848 A
- US P5801155 A
- US P5925517 A
- US P6492121 B
- JP 2650159 B
- JP 7114718 B
- JP 2500565 A
- WO 8810315 A
- JP 11046778 A
- JP 62265999 A
- JP 58040099 A

### Non-patent literature cited in the description


- Properties of chlamydia separated from the affected area of cervicitis. Pathogenic Microbe Detection Information Monthly Report (IASR. Infectious Disease Surveillance Center, August 2004, vol. 25, 204-205
- **READ T.D. et al.** *Nucleic Acid Research,* 2003, vol. 31, 2134-2147
- **WARREN R. et al.** *Journal of Clinical Microbiology,* 1993, vol. 31, 1663-1666
- **DOMEIKA M. et al.** *Journal of Clinical Microbiology,* 1994, vol. 32, 2350-2352
- **BAUWENS J.E. et al.** *Journal of Clinical Microbiology,* 1993, vol. 31, 3013-3106
- **CHERNESKY MAX A. et al.** *Journal of Clinical Microbiology,* 1994, vol. 32, 2682-2685
- **LEE H.H. et al.** *Lancet,* 1995, vol. 345, 213-216
- **BASSIRI M. et al.** *Journal of Clinical Microbiology,* 1995, vol. 33, 898-900
- **F. POLY et al.** *J. Bacteriology,* 2004, vol. 186 (14), 4781-4795
- *FEMS Microbiology Letters,* 1998, vol. 166, 63-70
- *Systematic and Applied Microbiology,* 2001, vol. 24, 109-112
- **VENTER et al.** *Science,* 16 February 2001, vol. 291 (5507), 1304-1351
- **D. M. MORRISON.** *Method in Enzymology,* 1979, vol. 68, 326-331
- *Nucleic Acids Res.,* 1986, vol. 14, 6115
- **BOOM R ; SOL CJ ; SALIMANS MM ; JANSEN CL ; WERTHEIM-VAN DILLEN PM ; NOORDAA J.** *J. Clin. Microbiol.,* March 1990, vol. 28 (3), 495-503
- **TSUGUNORI NOTOMI et al.** *Nucleic Acid Res.,* 2000, vol. 28, 63
- *Rinsho Byori,* November 2003, vol. 51 (11), 1061-1067
- **VENTER.** *Science,* 16 February 2001, vol. 291 (5507), 1304-1351

SEQUENCE LISTING

<110> Wako Pure Chemical Industries, Ltd.

<120> Process for detecting Chlamydophila caviae, as well as Chlamydophilia caviae detection method using the same

<130> 122370ep

<150> EP 09707584.0
<151> 2009-02-03

<150> JP 2008-029460
<151> 2008-02-08

<160> 40

<170> PatentIn version 3.3

<210> 1
<211> 1033
<212> DNA
<213> Chlamydophila caviae

<400> 1
```
tgtgttgttg aagtattctt tgttaacatt ttctttggta ctgcaatcgt taccttagct      60

ccggttacga ctgacaagtt aatcgaaata tttttagatt cccgctgctg atcctgaaca     120

gtaaatacat attcctgctc ttgaatctgc cccgtcccta atcgacctga tattactcca     180

gaagcatcca cggttaatcc attaggtaaa gctggagaaa ctgaaaccgt ataaggaggc     240

tctcctcctg taatttcaaa agcatgaata ttaacatggc tatcggttga tagaacacga     300

cggtataatt tctgagaaac tacaaaagga ggattcacta ctaaggaaaa tgttgctgag     360

gctctctgcg tgccattact atctccgaca tatacagtgt agcttgtttc tggaatccca     420

atttcagact gtacggtccc ggatattttc cctgatctag gatctaattg taactgatta     480

ggtaaagaag gctgaatcga ccattgctgt gtccctgaaa aggatgaagt caccgatatc     540

ggattttttc ctgcggtata agattttgct gtcactacaa ccttgggact atctacctgc     600

aatactggta aaaactctaa tcttgctgtt tgtgaatcgc tattcgaagt cgcactcact     660

gtagtttctc ctgaataggt attcgttaga gagactttaa cttgcccttg agaatctgaa     720

gtcaacgatg aggcatctag tgtagcgtga tttgacgctg ctaccgtaac aggaaaatta     780

ggaataggat tattatgcgc gtcaataatt ctgaatataa cttctgcttt atcttgacca     840

tttgccggct gattattttg taaaatcgta atggctcgcg aaacttgtgc tgtcgtctta     900

tcaggaacaa aaagaatcgg tattacatct acaacctgat taactatgac gtctacctcc     960

actagagagg cctctgttgt cttaattggg ggagtgagga ctttcccttg agaatctgtt    1020

aataatactt cat                                                       1033
```

<210> 2
<211> 989
<212> DNA

<213> Chlamydophila caviae

<400> 2
acctgaagat tttcctgaag gaacaaatat tcttgatctt cccgcctcct tattcttccc 60

agaaggagct ccaccacctc ttttcttaaa tcaacaagat attactgcgg caacacctac 120

atcccctcaa ccctccatcg catctcaaac gacgattgcc ccacaagcag cagatcagac 180

agcccctcaa caagatgata caataagcct accagaatcc ctggccgcta ttccacctct 240

cgaaacggct catatcatac gggaagttga tcaaacactc acagctcaag aatttttaaa 300

tagcgcttat ccaaatatgg atcatagtgc acttattcat ggcgctctta taaatgtaca 360

acttcaaggc attcctcttg aaaatagtga agatattctt ggtcttccag ctcttattgc 420

tttcccagat ctagttgctg gacaacccgt acgaccgaca ttcttagatc tcgacaatat 480

gccaagatct ctttccgctg atcaagagat agctcctcct ccccctactg cagcagatcc 540

catctctcca aatgatccta gatacatctt tctacagaat cacttccccg agttatctcc 600

cgaatactat agccaacata tcaatctgct tgcttcttta gctggagtag actctgacaa 660

ctttgacctt cttcaattac ctcttgaagt attcgttgaa gcaccagcac ctcaacctga 720

ctacagtcct atttctccag aagaagccca acaaagatac aatgatgttt ctgcagaaga 780

gcacgcaaga agaagtaacg attttatccg ttatcttata gaaaattcac cacaacgttg 840

gaccttctta aacagactga aaaataacat ctctgcatca acttcaagct taaccctgcg 900

tagagaatgg tttaaaatgt tggatgtaat atctaacaag acgagccctg aaatcacgga 960

tcgcgaagta caggatgttg cccgtgcat 989

<210> 3
<211> 929
<212> DNA
<213> Chlamydophila caviae

<400> 3
gatttaaaac caagatctaa cttattgttt tttagatctt ttcttctaac aaataatgtt 60

gtacaaactt tcttcgtaat taaaattaaa cggacgcgaa ataggatagg cttcacaaca 120

aaatgtgaag cctatttaga acatagtgat aagtgtctat ctatatactc gatgatcgat 180

tacgtgttct tggagtcagt tgtaaattta taacctctaa caaagtaatg cgtgcttctt 240

gctgtcggat acgcgctaaa gcatgaggaa gagagtctat gctatgatgt agttctacat 300

tctcataaga ttctacatcg cgttgtaagg ctgatataac ttcttcctta tcttctaata 360

aagtttctag ctcatgaata cgatcaagat ctacagcatg ttgctgactc acagaatctc 420

gtagaacaga aatctgttgt tcttgttctc gtatattttg attaaatatt tcttggtttt 480

gcaatagagt ttgtcggatt gttgtcaatt catcggcgtg tgatgtcacg tgttgcgaca 540

tctcattttg taaaaattcc attcgctctc taacggattt ctcctttgac tccatttctt 600

tttgatgatt attttgaagc tggttacgta gggatgtttg cctgataatc tctgcataac 660

```
attttctgtc ttgctcctcc aaaagttctg catgctcttt tcttagaagc tttattatgc    720

tttctaagtc aattatacgc tgatttcttg cttgtagaag cgttgcctgt tcttcagata    780

cgagaccttc tcggtctggc actctatgct cttgatcctc tagctgttga gcacgtagat    840

caacgagttc ttgctgtgca acagctagct gtgttgttag ctcagcactc cttgcctgtt    900

ctactaaaac tctttgggag acaactgct                                      929


<210>  4
<211>  1074
<212>  DNA
<213>  Chlamydophila caviae

<400>  4
cggcattccc ttggaattcg ttaatggtag cacctagatt aggaaaaaga aatgaaaatg     60

gggaaactcc ggtaacttta tgggtaacgc aaaaagctac ggtcgaacga gagatagctc    120

ttttacatca agctcgtgtt tttcccgatt ctatatcttg tcaacctgcg gacatctttt    180

ctcttgccca gcagacgcct ttaaaagctc tccctgccta tttcttaatt tatgcaggct    240

cttctgagac cacgtgcctt ttcgttcaaa atggagcagt tttagtttct cgttcatttt    300

gtaattctat cgagaaaaat agtgaggata tccttactac actagattac gtaaaagaaa    360

cctacccttc tgtagagctg acagctctac atactgtgca gcttccagaa ttattaaaaa    420

aatctttaga gcagaaactc tctttatctt tgattccctg tcaaacgatg acatttggtc    480

tagaagaaga ggaatgggca aattatggtg atgcgattac gacggcgtat catggagctt    540

ctcgagggac gctgactttt ccctacaatc cggtatttaa ctgtaaggag tcccagaaac    600

actggttaaa gcgctcggca tttacaatag gaaaactcgc gttattatct acagtacttg    660

taggtttagg ctctacttta aaactcgcct ctatttctca tcatgtgcgg gaacattttt    720

ctctagtgtg tcctgaaacc caaaaagttc ctagatcgct tcgtggtgta gaagaagcgc    780

tgcgtgctgc tatagcagaa aaaacgcgcc ctgaataccc ctatctacct acaattccaa    840

caaacaaaga aaccatgctg tttctttcct ctgtagcaca aagtacgcct tccgtgaaac    900

tttcgtattt ctgctactcc ctaacctctt tcccttcaca acagaatcct aaccgacact    960

acaaagcgca tgtttctgtt cggggtgaag gagatcccga agaagtgtca gagttcctcc   1020

gtaaactctc tctgcatccc aaactgtcca atattaccaa aacgttgtgc agcg         1074


<210>  5
<211>  1075
<212>  DNA
<213>  Chlamydophila caviae

<400>  5
ggaaagagag ttctctagaa tcatctatac gcaataaatt taagaattga ttgtgccttt     60

ctcctcttaa agcacataaa tagcgaatat catcatctat agggggacgt aaatatccac    120
```

atgtaatagg ctctattctt gatctctccg catcaagcgt ctcagctgca aatttcaaag 180

cctctgcccc tataggatct atgtccactc gcgcacctac tgacgcacct actgaggcat 240

cttcgggttc tcttggagtc tttaaacaaa ccttcactgc aatcaaaaat gcactgaatg 300

caatggctcc aagaacgata aaactcaggc caaggcctcc taaaatcaat aaaggaaggc 360

tagcacaact agcgagtcct aaagcaccaa gaattacgaa aattattggg attgcacatc 420

cgatgatact gccacaaacc acaagaggtt tggattggca taaacttttc ttcggctcaa 480

tattttctaa tgaaacctgt ctgcccaaga tgctatttat aaaagccata aaatcctaaa 540

aataaaaaac aaatcgaaaa attctaattt aaacaaaaat agaagtcaat ttttttattt 600

ttaagtcctc taaacaaaaa tatttagaaa atctttgtcc tacagttaat gaaattttat 660

ctttttgaag aatttcagtt taaattcgcg tgctgaagac attctagggg cataccttct 720

aagattacaa cacatcgggc tctgcatctc aaagctatgc attgctatat ttctaaaatg 780

accacgatat acgtaagcgc ttctaagcgt agcttttaca cagaaaacta ttgtattaat 840

atttttgttc cttataatgt tccaaacgat tttgacctcg tctactgtaa aacaacattg 900

cgtgattaga acattataaa aaatacatcg tatgcattcg ggttatgact cttccagaaa 960

aaacaacttc tgtcgcctct tcggctcaga ctcactccaa ttacacagca gcttctaatt 1020

cttcttcttt gcgtcaaaat aatactactc gcgcggcttc tttagatgaa aggct 1075

<210> 6
<211> 4474
<212> DNA
<213> Chlamydophila caviae

<400> 6
taggttgtta ttgaaaacaa tatttccctt atctgcagat agtgtgaggc tacaaccccc 60

gcctgctatc agtatagcgc ctccctcctg agcagagtta tttgtgaaga aaatatcgtt 120

attattgtaa atcgtgcata ccgcgccttt aatagctcct cctcgatatg aggaagagtt 180

attgtggaac gatatgattc ctttattatt tgctatagcg gtattttttg catcgatagc 240

tccaccatta ccatcgtgca tagacatggt tctattattt ttaaagatga ggtttccctg 300

attctctgag aatagacatg tagtatttac agagagagcc ccgccacttt tggattgatt 360

gttgttgaat acgatatctc ctaagttgtt gatgcagttg aagtcttggg ctcgtaaggc 420

tcctccttga ctatcaacag ctaaggttgc gttgtcgcaa aaaatacaag aggacttatt 480

gccggataaa tcaaatctct ctcctgacat gatcgcgcca ccttttgaat aggcggcgtt 540

tctaaggaaa tatatcggag cgttattatt tttgatatcg agaaatcggg agtttatagt 600

gccgccatca agtgaacgat tttggtcaag tgctgtgctg cgcgcgagat tatttgagaa 660

aacaatcact tgattgctat tggaaatctc taaatcacga ttagtgtaga ttcccgttcc 720

tgaacgtggt gatatattgc ctatcaggat aatgtgacct tgcgtatctt gaatgtataa 780

gttactacat tttacacttg ctccttctga atcagtagtc atttgggaaa tagcaaatat 840
atcttcgtaa ttctttagtg ataacgattc tgaagattgc gtttgtcctg tcaagttagt 900
tttctgtaag agaaactcta gagattgagg gatgtagagg gaattctcag aaggtagggg 960
agccagaaga actacttctc tagcatctat attataaaaa taggctaagg atattgagaa 1020
aataagattg taaataaact tatgtttcat gtgagttttc tcaatttata attttattaa 1080
aataccacgc gacttccagc atttatataa tgagacgttg tagatgaaga gaggtccaga 1140
tggtagttca agaaagcaac tatattatga aatagataag cagagtttct tccttcaacg 1200
tgtaaattat gtcgtgctac agcagttccc gaagatatcc aagatccatt gcttgccagc 1260
agtgttatta ggactttggg tttttgttca tacactaaag gttggtaggc aagttcgaat 1320
tcccagacac tgggtacgta ggagtctgca tgccaagaag ccagaattcc tacgggcaag 1380
gtgagagaaa ccaacggatg ttggttagag aattttctag aaaaatctcc agtttcattg 1440
atttgcgttt gttttgcccg taccccagcc aattgaatga agggtgtaac gtggtgattc 1500
aaaagaggaa gttttgtata acagcctata cgtgcgctta ggacatggtc gctaaattgt 1560
gtttctgatg ttttattttg tgcataaaaa ctttgcatat gatggtcccc ataaccatag 1620
cctaaggagg ctgtagttat aagatagtcg ttaaactggg gtgtttgcaa agttgcacct 1680
aacaggtagc tatgtgagct aactttattg ttagatgtat gttcttttat tttcccaatg 1740
agttgtgaga atgtgagtaa gaacttgtgc tgagtttttg aggagaaaag acattctccg 1800
ccatagccta aagatcgtga agaaaatcct aaaatttcat tttgatttct ttgacgggta 1860
tgtgatgcta atccgttacc ctctaggtaa aaagacgtag agtcgtttat caatgctggg 1920
taaaaagcca tggcattgcg agcagtttgt ctgagggtat tggcaacaag gtctccacga 1980
tgttgagggt tgactacata gcctgtaggc gtccagttag cataaaggta gcgatgtttt 2040
gagtttgttg tctctaacga tgaggttatc gaaggtgagg agacctcttc ccaataagga 2100
gtccaaatgc cttgatggcc ataatgctta tgcgcattta ttgcctgaac atcgaaatca 2160
tcaatggtta cagagttatt tgctggttca gagaggtgta agaggggaat tctatctatg 2220
ggttgggaaa ggtctacact atcataggga tcgctataat ttttattcca taaagatagg 2280
gatcccgata tcgaaatcct tgcgtcagta ttttcagtat agttgcctcc ggagcctcct 2340
gggtaaatcc aaacagtagg tgggctacta tcaggtttta aaatgtctgt aaggagaatg 2400
gcaacatttt ttaattgtaa gttactgtct ttttgcgaac tgctgttttc ttgggtttgc 2460
atgatcgcac gacttcctaa acagagatgc gtattatctt cgggagtgaa tttatatagc 2520
catagtatag cgccatcttc tacagagaca atgccgtgtt taattgtgag ttcaccctta 2580
tagctaattt gaagattttt gatgctctga ttagctagct ttaattgagt gctcgctgct 2640
gagaatacta ccgctcctgt atgtttatct tcaggattaa tgataaaaga ggatagaccg 2700
ccagtgttca ttctatcaaa gagatagata tgttggttgc tggaagcccc taaagatagg 2760

gagtggttcg agctgaaatg catagcggat ctccaaaccc ccattctatc tatcgcaata 2820
ttgttatcaa aaattacatc gccataatct gcagatatgg tggtagttcc gttatttgtt 2880
tgtccataac atgcagctcc ccagcaagaa gtattattta gaaagtagac aggcccgtta 2940
ttttggatag tgaggtgttg ataggagaga gcgcctccag catgagccga ggagtttcca 3000
gaaaatagca caggttgttt attgttttcg atgacaatag ttgggctatt aattgctccc 3060
ccatttcctt ggttactggt gttagaagca acattatgag aaaaggtgat aggcgcctga 3120
ttatcaacaa ttcttgtcga ggttgtagaa caaatcgccc cgccttttcc agatttattc 3180
tcactaaatg taatttttcc acgattgcgg gcaaattcac aagagactgc agaaatcgct 3240
cctccaaagc tgtctgcatt ggtagttcta ttattatcga agtgtatgga tgcagaatta 3300
ttaaagaacc tgcacatttc cgtagaggca atggctcctc cttttacttt agttaaattg 3360
gaggaaaaaa gtgtctcttt gttaagggaa gcttcaaaat tttcgcttg tatagcaccg 3420
ccagtaccac tagagctatt tgtttgattg agagtggtgt tttgataaaa aatgatacga 3480
gagtttttg ataggattac gctttcagca gaatagattg ctccaccaca caaggttgat 3540
ttgttatctc caaagaaaat aggttgggta ttgtttataa gactacaaga gcgacaattg 3600
atcacaccgc catcggttgt attcaatttg catataaaca ttttaggttg cttatcgttg 3660
gcgataacaa tattttgagc ggcgttgtgc agtaatccgt agttcttctc gaaatcatct 3720
tgatttacgc gtgttaatgg aaaatcaaga gcagaaagta gcttagagat ctcttcaggg 3780
attttaatgt cttcactata gataatgctt tgaacacaag cagataggat cagagagcag 3840
agaagagacc tagggaacat atttaaaaaa tgtagttgtg atatgatttt ttcccctata 3900
aaggacttct tctatctttg tctagaagtt atagacgctg atctttaaga gtgcgtgata 3960
aatgaaaaag gtttgttttg aataaaacaa acctttagg gtgatgtatt ttaaagagtt 4020
aaaaatacac tgcgagattg agaacctcct aaaacctatc ttagtgattt tagaaagaga 4080
gttttcctcc agcgagcagg taatggctgc gtgtagatga ggataaatca cattgatagt 4140
ctacatgcat gttgagatat ttgagtactt gtgtttcgtt tctaagattt acagaaacag 4200
cacggcggct cacatttgta ggggtagaaa cccaagttcc atcatctgca actaatttgg 4260
aacttactac agggagttga cgttgagttg aaggcttata ggcaacttct aattcccaaa 4320
ttgatgggaa acgtccgtga aaagctaact gactatgcaa tccgaaaggc aggctagtat 4380
caagaaactc atcgaatttt ggagagcctg agaaagtgcg tacacgagcg ccttgttctg 4440
tgaatggatc atgtcttgaa agaacagcct gaag 4474

<210> 7
<211> 19
<212> DNA
<213> Artificial

<220>
<223> oligonucleotide primer

<400> 7
atctgccccg tccctaatc 19

<210> 8
<211> 20
<212> DNA
<213> Artificial

<220>
<223> oligonucleotide primer

<400> 8
accgtcgtgt tctatcaacc 20

<210> 9
<211> 21
<212> DNA
<213> Artificial

<220>
<223> oligonucleotide primer

<400> 9
tctgcgtgcc attactatct c 21

<210> 10
<211> 21
<212> DNA
<213> Artificial

<220>
<223> oligonucleotide primer

<400> 10
cttcatcctt ttcagggaca c 21

<210> 11
<211> 20
<212> DNA
<213> Artificial

<220>
<223> oligonucleotide primer

<400> 11
tcttcccgcc tccttattct 20

<210> 12
<211> 18
<212> DNA
<213> Artificial

<220>
<223> oligonucleotide primer

<400> 12
gctgcttgtg gggcaatc 18

<210> 13
<211> 20
<212> DNA
<213> Artificial

<220>
<223> oligonucleotide primer

<400> 13
cagaatcact tccccgagtt 20

<210> 14
<211> 22
<212> DNA
<213> Artificial

<220>
<223> oligonucleotide primer

<400> 14
cattgtatct ttgttgggct tc 22

<210> 15
<211> 19
<212> DNA
<213> Artificial

<220>
<223> oligonucleotide primer

<400> 15
cgtagggatg tttgcctga 19

<210> 16
<211> 20
<212> DNA
<213> Artificial

<220>
<223> oligonucleotide primer

<400> 16
tagagtgcca gaccgagaag 20

<210> 17
<211> 22
<212> DNA
<213> Artificial

<220>
<223> oligonucleotide primer

<400> 17
gcgaaatagg ataggcttca ca 22

<210> 18
<211> 23
<212> DNA

<213> Artificial

<220>
<223> oligonucleotide primer

<400> 18
gcacgcatta ctttgttaga ggt 23

<210> 19
<211> 20
<212> DNA
<213> Artificial

<220>
<223> oligonucleotide primer

<400> 19
ttcgttcaaa atggagcagt 20

<210> 20
<211> 25
<212> DNA
<213> Artificial

<220>
<223> oligonucleotide primer

<400> 20
cagggaatca aagataaaga gagtt 25

<210> 21
<211> 23
<212> DNA
<213> Artificial

<220>
<223> oligonucleotide primer

<400> 21
aaaaagaaat gaaaatgggg aaa 23

<210> 22
<211> 20
<212> DNA
<213> Artificial

<220>
<223> oligonucleotide primer

<400> 22
gctgggcaag agaaaagatg 20

<210> 23
<211> 21
<212> DNA
<213> Artificial

<220>
<223> oligonucleotide primer

<400> 23
tgattgtgcc tttctcctct t 21

<210> 24
<211> 20
<212> DNA
<213> Artificial

<220>
<223> oligonucleotide primer

<400> 24
gcctcagtag gtgcgtcagt 20

<210> 25
<211> 20
<212> DNA
<213> Artificial

<220>
<223> oligonucleotide primer

<400> 25
cgggttctct tggagtcttt 20

<210> 26
<211> 20
<212> DNA
<213> Artificial

<220>
<223> oligonucleotide primer

<400> 26
tgtggtttgt ggcagtatca 20

<210> 27
<211> 20
<212> DNA
<213> Artificial

<220>
<223> oligonucleotide primer

<400> 27
atggctgcgt gtagatgagg 20

<210> 28
<211> 21
<212> DNA
<213> Artificial

<220>
<223> oligonucleotide primer

<400> 28
cagatgatgg aacttgggtt t 21

<210> 29

<211> 160
<212> DNA
<213> Artificial

<220>
<223> oligonucleotide probe

<400> 29
atctgccccg tccctaatcg acctgatatt actccagaag catccacggt taatccatta 60

ggtaaagctg gagaaactga aaccgtataa ggaggctctc ctcctgtaat ttcaaaagca 120

tgaatattaa catggctatc ggttgataga acacgacggt 160

<210> 30
<211> 165
<212> DNA
<213> Artificial

<220>
<223> oligonucleotide probe

<400> 30
tctgcgtgcc attactatct ccgacatata cagtgtagct tgtttctgga atcccaattt 60

cagactgtac ggtcccggat attttccctg atctaggatc taattgtaac tgattaggta 120

aagaaggctg aatcgaccat tgctgtgtcc ctgaaaagga tgaag 165

<210> 31
<211> 135
<212> DNA
<213> Artificial

<220>
<223> oligonucleotide probe

<400> 31
tcttcccgcc tccttattct tcccagaagg agctccacca cctcttttct taaatcaaca 60

agatattact gcggcaacac ctacatcccc tcaaccctcc atcgcatctc aaacgacgat 120

tgccccacaa gcagc 135

<210> 32
<211> 190
<212> DNA
<213> Artificial

<220>
<223> oligonucleotide probe

<400> 32
cagaatcact tccccgagtt atctcccgaa tactatagcc aacatatcaa tctgcttgct 60

tctttagctg gagtagactc tgacaacttt gaccttcttc aattacctct tgaagtattc 120

gttgaagcac cagcacctca acctgactac agtcctattt ctccagaaga agcccaacaa 180

agatacaatg 190

<210> 33
<211> 180
<212> DNA
<213> Artificial

<220>
<223> oligonucleotide probe

<400> 33
cgtagggatg tttgcctgat aatctctgca taacattttc tgtcttgctc ctccaaaagt 60

tctgcatgct cttttcttag aagctttatt atgctttcta agtcaattat acgctgattt 120

cttgcttgta gaagcgttgc ctgttcttca gatacgagac cttctcggtc tggcactcta 180

<210> 34
<211> 140
<212> DNA
<213> Artificial

<220>
<223> oligonucleotide probe

<400> 34
gcgaaatagg ataggcttca caacaaaatg tgaagcctat ttagaacata gtgataagtg 60

tctatctata tactcgatga tcgattacgt gttcttggag tcagttgtaa atttataacc 120

tctaacaaag taatgcgtgc 140

<210> 35
<211> 200
<212> DNA
<213> Artificial

<220>
<223> oligonucleotide probe

<400> 35
ttcgttcaaa atggagcagt tttagtttct cgttcatttt gtaattctat cgagaaaaat 60

agtgaggata tccttactac actagattac gtaaaagaaa cctacccttc tgtagagctg 120

acagctctac atactgtgca gcttccagaa ttattaaaaa aatctttaga gcagaaactc 180

tctttatctt tgattccctg 200

<210> 36
<211> 149
<212> DNA
<213> Artificial

<220>
<223> oligonucleotide probe

<400> 36
aaaaagaaat gaaaatgggg aaactccggt aactttatgg gtaacgcaaa aagctacggt 60

cgaacgagag atagctcttt tacatcaagc tcgtgttttt cccgattcta tatcttgtca 120

acctgcggac atcttttctc ttgcccagc 149

<210> 37
<211> 191
<212> DNA
<213> Artificial

<220>
<223> oligonucleotide probe

<400> 37
tgattgtgcc tttctcctct taaagcacat aaatagcgaa tatcatcatc tatagggga 60

cgtaaatatc cacatgtaat aggctctatt cttgatctct ccgcatcaag cgtctcagct 120

gcaaatttca aagcctctgc ccctatagga tctatgtcca ctcgcgcacc tactgacgca 180

cctactgagg c 191

<210> 38
<211> 200
<212> DNA
<213> Artificial

<220>
<223> oligonucleotide probe

<400> 38
cgggttctct tggagtcttt aaacaaacct tcactgcaat caaaaatgca ctgaatgcaa 60

tggctccaag aacgataaaa ctcaggccaa ggcctcctaa aatcaataaa ggaaggctag 120

cacaactagc gagtcctaaa gcaccaagaa ttacgaaaat tattgggatt gcacatccga 180

tgatactgcc acaaaccaca 200

<210> 39
<211> 146
<212> DNA
<213> Artificial

<220>
<223> oligonucleotide probe

<400> 39
atggctgcgt gtagatgagg ataaatcaca ttgatagtct acatgcatgt tgagatattt 60

gagtacttgt gtttcgtttc taagatttac agaaacagca cggcggctca catttgtagg 120

ggtagaaacc caagttccat catctg 146

<210> 40
<211> 27
<212> DNA
<213> Artificial

<220>
<223> oligonucleotide probe

<400> 40
tcaacaagat attactgcgg caacacc 27